# EUROPEAN PATENT SPECIFICATION

(11) **EP 4 728 970 B1**
(45) Date of publication and mention of the grant of the patent: **23.09.2026**
(21) Application number: 25204883.0
(22) Date of filing: 26.09.2025
(51) Int. Cl.: A61B 5/00, A61B 5/339

(54) **SYNCHRONIZED DISPLAY OF REAL-TIME PHYSIOLOGIC SIGNALS OBTAINED USING IMPLANTABLE AND EXTERNAL DEVICES THAT WIRELESS COMMUNICATE WITH ONE ANOTHER**
SYNCHRONISIERTE ANZEIGE PHYSIOLOGISCHER ECHTZEITSIGNALE AUS IMPLANTIERBAREN UND EXTERNEN VORRICHTUNGEN, DIE DRAHTLOS MITEINANDER KOMMUNIZIEREN
AFFICHAGE SYNCHRONISÉ DE SIGNAUX PHYSIOLOGIQUES EN TEMPS RÉEL OBTENUS À L'AIDE DE DISPOSITIFS IMPLANTABLES ET EXTERNES QUI COMMUNIQUENT SANS FIL LES UNS AVEC LES AUTRES

(30) Priority: 17.10.2024 US 202463708576 P; 24.09.2025 US 202519339168
(43) Date of publication of application: 22.04.2026
(73) Proprietor: Pacesetter, Inc., Sylmar, CA 91342 (US)
(72) Inventor: Deshmukh, Nilesh, Sylmar, CA 91342 (US); Bisen, Pulkit, Sylmar, CA 91342 (US); Singh, Tejpal, Sylmar, CA 91342 (US); Young, Chao-Wen, Sylmar, CA 91342 (US); Li, Xinghon, Sylmar, CA 91342 (US); Wu, Yongjian, Sylmar, CA 91342 (US)
(74) Representative: Barker Brettell Sweden AB

(56) References cited:
- US-A1- 2016 059 024
- US-A1- 2017 196 457
- US-B2- 10 686 878

## Description

### TECHNICAL FIELD

Embodiments of the present technology described herein relate generally to systems, devices, and methods including or for use with an implantable medical device (IMD) and an external device (ED) that are configured to wirelessly communicate with one another using a wireless communication technology, such as, but not limited to, Bluetooth^{®} Low Energy (BLE) technology.

### BACKGROUND

An implantable medical device (IMD) may utilize wireless telemetry, such as Bluetooth^{®} Low Energy (BLE) telemetry, to wirelessly communicate with an external device (ED), such as a bedside monitor and/or a programmer. It is often desirable for such an ED to provide a scrolling display of real-time or near-real-time physiologic signal data that is obtained by the ED, as well as by the IMD. For example, the ED may receive two types of real-time signal data for monitoring a patient's cardiac rhythm, one of which can be data corresponding to surface electrocardiogram (ECG) signal obtained using skin electrodes and the other of which can be data corresponding to an electrogram (EGM) signal sensed using implanted electrodes, but not limited thereto. The ECG signal can be sensed from the patient's body using one or more directly or communicatively coupled skin electrodes, while the EGM signal can be sensed using electrodes of or communicatively coupled to the IMD and data indicative thereof can be transmitted wirelessly from the IMD to the ED using BLE or another type of wireless telemetry.

However, difficulties may arise when the ED attempts to display the ECG and EGM signals simultaneously and in real-time or near-real-time. Even though both types of real-time signals (i.e., the real-time ECG and EGM signals) are generated at the same time from the same heart, the sample data of ECG and EGM signals follow different paths as the data travel from the patient to the ED. The different paths introduce different delays that cause the ECG and EGM sample data to arrive at the ED out-of-sync with one another. If left uncorrected, the out-of-sync data are presented out of sync with one another to a user at a display of (or communicatively coupled to) the ED. More specifically, the displayed ECG and the displayed EGM will be out-of-sync with one another if the out-of-sync data is left uncorrected. This can result in misleading information being presented on the display to a person (e.g., a clinician or other medical practitioner) if the ECG and EGM data are not presented in a coherent in-synch manner.

The dyssynchrony of the ECG and EGM data becomes even more prevalent when utilizing BLE technology to wireless transmit the EGM data (or other type of physiologic signal data) from the IMD to the ED. An inherent characteristic of the BLE protocol is the transmission of data in short bursts, rather than in a constant stream. While the BLE protocol seeks to utilize a maximum available BLE bandwidth, the BLE protocol is not optimized to provide real-time data, such as EGM data, in a manner that affords a smooth in-sync scrolling display of real-time EGM traces. Also, the IMD introduces a certain amount of processing delay to process the EGM data and a communication delay to wirelessly transmit the EGM data to the ED. The aforementioned processing delay and communication delay introduce some constant and some indeterministic delays which can cause the display of real-time EGM data to be delayed and staggered with reference to corresponding ECG data.

Conventional approaches for attempting to synchronize different types of physiologic signal data (e.g., ECG data and EGM data), which are sensed using different sense electrodes and/or other types of sensors, experience design challenges that do not compensate for the various delays and do not convert the short bursts of EGM data into a constant stream of EGM data at a desired rate. Conventional approaches also do not cause display of ECG and EGM data synchronized in a manner that enables a clinician to perform proper diagnosis.

To achieve sufficient EGM quality, a sampling rate of a sensed EGM signal may preferably be within the range of about 250 to 300 samples per second, with a minimum rate of 128 samples per second (i.e., with a minimum of the sensed EGM signal being sampled once per 7.81 milliseconds). The transmission of EGM sample data, from an IMD to an ED, using BLE may involve software support that may act on interrupts to trigger data transfer from an EGM hardware buffer to a BLE transceiver through a serial to parallel interface (SPI). The BLE transceiver can utilize the BLE protocol stack to transmit data through a BLE interface. Separately transmitting each sample of EGM data, e.g., at least every 7.81 milliseconds, can cause a respective data transmission interrupt once every at least 7.81 milliseconds, which can cause high current consumption due to the software processing overhead.

To reduce the power consumption, the EGM data can instead be accumulated over multiple sampling periods as a bulk EGM packet by hardware of the IMD before triggering an interrupt for EGM packet transmission. The ED may have a buffer that is large enough to store enough bulk EGM packets for a spacing algorithm before streaming EGM samples to a display, e.g., as disclosed in U.S. Patent No. 10,686,878, titled "METHOD AND DEVICE FOR MANAGING DISPLAY OF MULTIPLE DATA STREAMS." The aforesaid mechanism, which was disclosed in the '878 patent, works well when there is relatively low air interference. However, when the air interference is high and causes many BLE packet retransmissions and/or BLE telemetry breaks, a firmware application may need to drop the incoming EGM data and/or re-start an EGM hardware interface to recover from a hardware error, such as a hardware buffer overflow. In scenarios like these, the relative positions of the newly arrived EGM packets to the earlier received EGM packets are lost. This may cause the ED to terminate the EGM streaming and re-start another synchronization process by waiting for enough consecutive samples in a predefined time slot to buffering samples to stream in synchronization along with the surface ECG data. Accordingly, it should be appreciated that the aforementioned mechanism does not guarantee to synchronize streaming of the surface ECG data and the real-time EGM data when medium or high air interference is present, which may result in errors of hardware, or when the real-time EGM stops and then starts again.

Accordingly, it can be appreciated that it would be desirable to provide new techniques for enabling synchronous display of, for instance, a surface ECG sensed using surface electrodes and an EGM sensed using implanted electrodes. More generally, it would be desirable to provide new techniques for enabling synchronous display of first and second sensed physiologic signals, where the signal sample data for one of the physiologic signals is obtained by an IMD, and the signal sample data for the other one of the physiologic signals is obtained by an ED.

US 2017/196457 A1 discloses a system including an implantable medical device configured to sense a sync signal and sense physiological parameters to obtain a physiological signal. In response to sensing the sync signal, the implantable medical device is configured to generate a sync-stamped physiological signal. In certain embodiments, a method includes receiving a first physiological signal coupled with a sync signal; receiving a second physiological signal coupled with the sync signal; and, using the sync signal, synchronizing in time the first and second physiological signals.

US 2016/059024 A1 discloses a cardiac rhythm management system including a first implantable device such as a defibrillator and a second implantable device such as a leadless cardiac pacemaker. A programmer is configured to receive and display heart data emanating from the implantable defibrillator and from the leadless cardiac pacemaker. The heart data emanating from the leadless cardiac pacemaker is displayed in temporal alignment with the heart data emanating from the implantable defibrillator.

### BRIEF SUMMARY

Certain embodiments of the present technology are directed to an ED configured to wirelessly communicate with an IMD, wherein the ED comprises a memory, a transceiver that enables the ED to wirelessly communicate with the IMD, and at least one controller coupled to the transceiver and the memory.

In accordance with certain embodiments, while a wireless connection is in a process of being established between the ED and the IMD, the at least one controller is/are configured to: control the transceiver to transmit a connection request packet to the IMD in response to receiving an advertising packet from the IMD to thereby enable the wireless connection to be established between the ED and the IMD; control the transceiver to receive from the IMD an IMD anchor point transmitted by the IMD in response to the IMD receiving the connection request packet from the ED; save in the memory the IMD anchor point; and determine an ED anchor point and also save in the memory the ED anchor point.

In accordance with certain embodiments, while the wireless connection is established between the ED and the IMD, the at least one controller is/are configured to: control the transceiver to receive at least some of a plurality of data packets from the IMD, wherein each data packet of the plurality of data packets that are received from the IMD includes a portion of physiologic signal data and a respective time stamp included by the IMD; determine, for each said data packet of the data packets received from the IMD, a respective time when the data packet was transmitted by the IMD from a perspective of the ED based on the ED anchor point that is stored in the memory, the IMD anchor point that is stored in the memory, and the respective time stamp included in the data packet received from the IMD; obtain further physiologic signal data representative of a further physiologic signal sensed using one or more skin electrodes or a non-implanted sensor; and use the respective times, determined for the data packets received from the IMD, to cause co-displaying of respective segments of a physiologic signal that are represented by the data packets along with the further physiologic signal such that they are synchronized with one another on a display of or communicatively coupled to the ED.

In accordance with certain embodiments, the IMD anchor point and the ED anchor point correspond to when the wireless connection is established between the ED and the IMD. In other words, the IMD anchor point and the ED anchor point are indicative of a point in time when the wireless connection is established between the ED and IMD.

In accordance with certain embodiments, the at least one controller of the ED is/are configured to determine the respective time when a said data packet was transmitted by the IMD from a perspective of the ED, for each said data packet of the data packets received by the ED, using an equation as follows: RT_{DATA} = ANCHOR_{ED} + TIME_STAMP_{IMD} - ANCHOR_{IMD}, where RT_{DATA} is the respective time when the data packet was transmitted by the IMD from the perspective of the ED, which respective time is determined by the ED, ANCHOR_{ED} is the ED anchor point stored by the ED, TIME_STAMP_{IMD} is the IMD time stamp included in the data packet received by the ED, and ANCHOR_{IMD} is the IMD anchor point stored by the ED.

In accordance with certain embodiments, the ED further comprises one or more buffers, and the at least one controller of the ED is/are further configured to control the one or more buffers to cause the further physiologic signal and the physiologic signal be synchronized with one another when they are co-displayed on the display of or communicatively coupled to the ED.

In accordance with certain embodiments, the physiologic signal comprises one of an EGM or a subcutaneous ECG, and the further physiologic signal, that is co-displayed and synchronized with the one of the EGM or the subcutaneous ECG, comprises an ECG sensed using the one or more skin electrodes.

Certain embodiments of the present technology are directed to a system including the ED according to one of the embodiments summarized above, and further comprising the IMD, wherein the IMD comprises a memory of the IMD, a transceiver of the IMD that enables the IMD to wirelessly communicate with the ED, and at least one controller of the IMD coupled to the transceiver of the IMD and the memory of the IMD.

In accordance with certain embodiments, while the wireless connection is established between the transceiver of the ED and the transceiver of the IMD, the at least one controller of the IMD is/are configured to: obtain the physiologic signal data representative of the physiologic signal for the patient within which the IMD is implanted; and control the transceiver of the IMD to transmit the plurality of data packets to the ED, wherein each data packet, of the plurality of data packets that are transmitted by the IMD to the ED, includes a portion of the physiologic signal data and a respective time stamp specifying when the data packet is transmitted by the IMD from a perspective of the IMD.

In accordance with certain embodiments, while the wireless connection is in the process of being established between the transceiver of the ED and the transceiver of the IMD, the at least one controller of the IMD is/are configured to control the transceiver of the IMD to transmit an advertising packet, receive the connection request packet from the ED within a receive window following when the IMD transmits the advertising packet, and in response thereto, transmit to the ED a connection response packet including the IMD anchor point.

In accordance with certain embodiments, the IMD includes a real-time clock (RTC), the IMD anchor point comprises a value of the RTC of the IMD when a connection response packet is transmitted by the IMD in response to the IMD receiving the connection request packet from the ED, and the respective time stamp of each said data packet of the data packets received by the ED from the IMD comprises a respective value of the RTC of the IMD when the data packet is transmitted by the IMD.

In accordance with certain embodiments, the ED includes an RTC, and the ED anchor point comprises a value of the RTC of the ED when the ED receives, from the IMD, the connection response packet.

In accordance with certain embodiments, the physiologic signal data, that is obtained by the IMD, is determined based on a real-time physiologic signal that is sensed by the IMD or by a further implantable device communicatively coupled to the IMD. The further physiologic signal data, that is obtained by the ED, is determined based on a further real-time physiologic signal that is sensed by the ED or by a further external device communicatively coupled to the ED. In certain such embodiments, the respective segments of the physiologic signal and the further physiologic signals are co-displayed in real-time or near-real-time such that they are synchronized with one another.

Certain embodiments of the present technology are directed to a method for synchronous display of signal segments, the method comprising: an ED transmitting a connection request packet to an IMD in response to receiving an advertising packet from the IMD to thereby enable establishment of a wireless connection between the ED and the IMD; the ED receiving, during a process of establishing the wireless connection between the ED and the IMD, an IMD anchor point transmitted by the IMD in response to the IMD receiving the connection request packet; and the ED storing the IMD anchor point and an ED anchor point. The method further comprises: the ED receiving, while the wireless connection is established between the ED and the IMD, a plurality of data packets, each of which includes a portion of physiological signal data for a patient obtained by the IMD and a respective IMD time stamp specifying when the data packet is transmitted by the IMD; and the ED determining, for each said data packet received by the ED, a respective time when the data packet was transmitted by the IMD from a perspective of the ED based on the ED anchor point that is stored by the ED, the IMD anchor point that is stored by the ED, and the IMD time stamp included in the data packet received by the ED. The method additionally comprises: the ED obtaining further physiologic signal data representative of a further physiologic signal sensed using one or more skin electrodes or a non-implanted sensor; and the ED using the respective times, determined for the data packets received by the ED from the IMD, to cause co-displaying of respective segments of a physiologic signal that are represented by the data packets along with the further physiologic signal such that they are synchronized with one another.

In accordance with certain embodiments, the ED determining the respective time when a said data packet was transmitted by the IMD from the perspective of the ED, is performed by the ED for each said data packet of the data packets received by the ED, using an equation as follows: RTDATA = ANCHORED + TIME_STAMPIMD - ANCHORIMD, where RTDATA is the respective time when the data packet was transmitted by the IMD from the perspective of the ED, which respective time is determined by the ED, ANCHORED is the ED anchor point stored by the ED, TIME_STAMPIMD is the IMD time stamp included in the data packet received by the ED, and ANCHORIMD is the IMD anchor point stored by the ED.

In accordance with certain embodiments, the IMD and the ED each includes a respective RTC, the IMD anchor point received by the ED comprises a value of the RTC of the IMD when a connection response packet is transmitted by the IMD in response to the IMD receiving the connection request packet from the ED, and the respective time stamp of each said data packet of the data packets received by the ED from the IMD comprises a value of the RTC of the IMD when the data packet is transmitted by the IMD.

In accordance with certain embodiments, the ED anchor point comprises a value of the RTC of the ED when the ED receives, from the IMD, the connection response packet.

In accordance with certain embodiments, the ED further comprises one or more buffers and the method includes the ED controlling the one or more buffers to cause the further physiologic signal and the physiologic signal be synchronized with one another when they are co-displayed.

In accordance with certain embodiments, while the wireless connection is in the process of being established between the ED and the IMD, the method also includes: the IMD transmitting an advertising packet; and the IMD receiving the connection request packet from the ED within a receive window following the transmitting the advertising packet and in response thereto the IMD transmitting to the ED the connection response packet including the IMD anchor point.

In accordance with certain embodiments, while the wireless connection is established between the ED and the IMD, the method also includes: the IMD obtaining the physiologic signal data for the patient within which the IMD is implanted; and the IMD transmitting the plurality of data packets to the ED, each of which includes a portion of the physiologic signal data and a respective IMD time stamp specifying when the data packet is transmitted by the IMD.

This summary is not intended to be a complete description of the embodiments of the present technology. Other features and advantages of the embodiments of the present technology will appear from the following description in which the preferred embodiments have been set forth in detail, in conjunction with the accompanying drawings and claims.

### BRIEF DESCRIPTION OF THE DRAWINGS

FIG. 1 illustrates a high level block diagram of an example system including an IMD and an ED with which embodiments of the present technology can be used.
FIG. 2 illustrates a high level block diagram of an example embodiment of the IMD introduced in FIG. 1.
FIG. 3 illustrates a high level block diagram of an example embodiment of the ED introduced in FIG. 1.
FIG. 4 illustrates a high level flow diagram used to describe a method according to an embodiment of the present technology for use with a system including an ED and an IMD configured to wirelessly communicate with one another, e.g., using BLE.
FIG. 5 is a high level block diagram of a memory including various portions used to store various types of data in accordance with an embodiment of the present technology.
FIG. 6A shows examples of an EGM and an ECG displayed out-of-sync with one another.
FIG. 6B shows the EGM and the ECG, originally shown in FIG. 6A, displayed using an embodiment of the present technology such that both signals are in-sync with one another.

### DETAILED DESCRIPTION

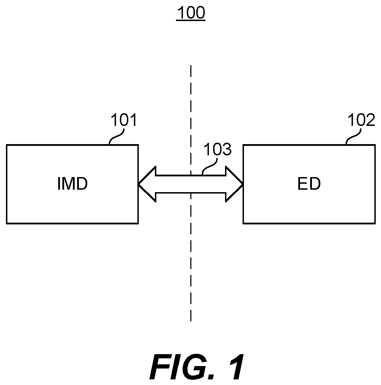

Embodiments of the present technology described herein relate to methods, systems, and devices that enable synchronous display of, for instance, a surface ECG sensed using surface electrodes and an EGM sensed using implanted electrodes. More generally, embodiments of the present technology described herein enable synchronous co-display of first and second sensed physiologic signals, where the signal sample data for one of the physiologic signals is obtained by an IMD using implanted electrodes or an implanted sensor, and the signal sample data for the other one of the physiologic signals is obtained by an ED using non-implanted electrodes or a non-implanted sensor. However before providing additional details of such embodiments of the present technology, examples systems and devices with which embodiments of the present technology can be used are described below with reference to FIGS. 1, 2, and 3.

### Example System

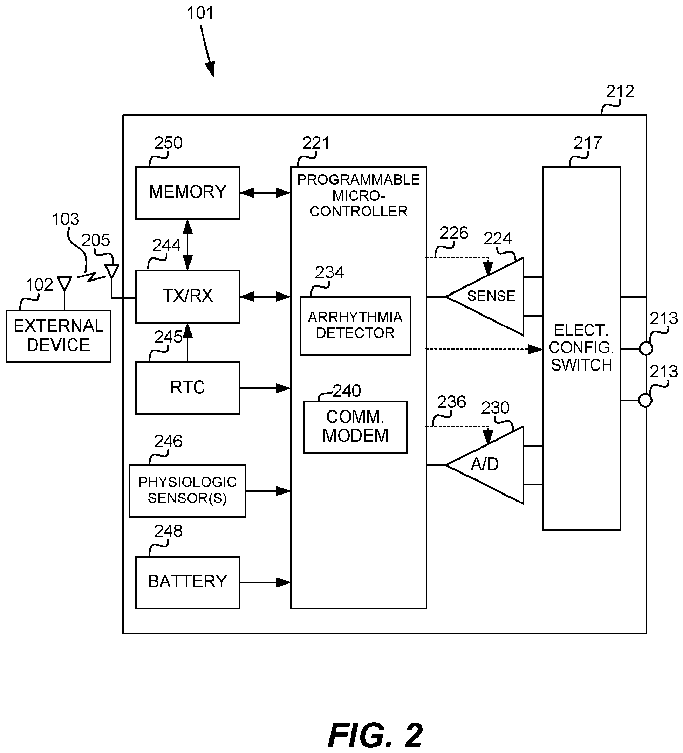

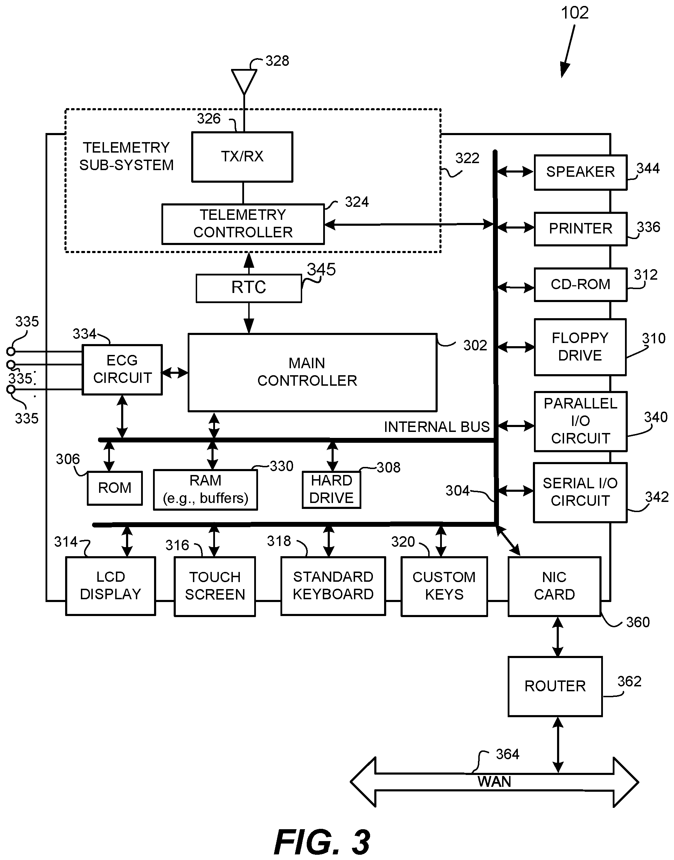

Referring to FIG. 1, a system 100 is shown as including an implantable medical device (IMD) 101 and an external device (ED) 102, that are configured to wirelessly communicate with one another over a wireless link 103. In FIG. 1, the block shown to the left of the vertical dashed line is implanted within a patient, and the block shown to the right of the vertical dashed line is external to (i.e., not implanted within) the patient. The system 100 is an example of a system with which embodiments of the present technology can be used. FIG. 2 illustrates a block diagram of an example embodiment of the IMD 101 introduced in FIG 1, and FIG. 3 illustrates a block diagram of an example embodiment of the ED 102 introduced in FIG. 1.

The IMD 101 can be an implantable pacemaker and/or an implantable cardioverter defibrillator (ICD) that includes and/or is coupled to one or more leads having one or more electrodes that are implanted within and/or near to a patient's heart. Optionally, the IMD 101 may be a leadless pacemaker (LP) that is implanted in or on a cardiac chamber, wherein the LP includes at least two electrodes that can be used to sense an EGM. It would also be possible for the IMD 101 to be an insertable cardiac monitor (ICM) that includes electrodes used to sense an EGM. Such an IMD can additionally, or alternatively, include one or more sensors that enables the IMD to sense other types of physiological signals besides an EGM. As discussed in additional detail below with reference to FIG. 3, the ED 102 can be, for example, a bedside or other type of external monitor, or a programmer, but is not limited thereto.

The IMD 101 may sense an EGM using one or more implanted electrodes provided on the housing of the IMD 101 and/or on one or more leads that extend from the housing. The sensed EGM can be associated with one or more paced and/or sensed cardiac events. The IMD 101 may additionally, or alternatively, sense other types of physiologic signals in addition to, or instead of, an EGM. Examples of physiologic signals that the IMD 101 may sense include, but are not limited to, an EGM, a subcutaneous ECG, a pressure signal, a cardiac impedance signal, a respiratory signal, a photoplethysmography (PPG) signal, an impedance plethysmography (IPG) signal, a temperature signal, a flow signal, and/or the like. For much of the following discussion, it will be assumed that the physiologic signal that is sensed by the IMD 101 is an EGM signal. However, it should be understood that embodiments of the present technology can also be used with other types of physiologic signals, some examples of which were just provided.

The IMD 101 can process a sensed physiologic signal (e.g., a sensed EGM signal) to produce physiologic signal sample data (e.g., EGM sample data) and can transmit the physiologic signal sample data (e.g., EGM sample data) over the wireless link 103 to the ED 102. In accordance with certain embodiments, the physiologic signal sample data (e.g., EGM sample data) is transmitted over the wireless link 103 in accordance with a wireless protocol, such as the Bluetooth^{®} Low Energy (BLE) protocol, where the IMD 101 transmits the sample data in short bursts in an intermittent manner as defined by the BLE protocol. The BLE protocol defines a burst type of data transfer and thus the sample data is transmitted with uneven throughput. The ED 102 receives a stream of sample data over the wireless link 103 with a corresponding throughput. While the wireless communication between the IMD 101 and the ED is often described herein as being implemented using BLE, the wireless communication can alternatively be implemented using other types of radio frequency (RF) communication besides BLE, such as with classic Bluetooth^{®}, ZigBee^{®}, Wireless Universal Serial Bus (USB), and Medical Implant Communication Service (MISC), but not limited thereto. It is also possible that other types of wireless communication besides RF communication be used, such as inductive communication or conductive communication.

It is noted that the terms "ECG" and "ECG signal" are used interchangeably herein. Similarly, it is noted that the term "EGM" and "EGM signal" are used interchangeably herein. Further, it is noted that the term "ECG data" and "ECG sample data" are used interchangeably herein. Similarly, it is noted that the term "EGM data" and "EGM sample data" are used interchangeably herein.

### Example Implantable Medical Device (IMD)

FIG. 2 shows an example block diagram of the IMD 101. The IMD 101 has a housing 212 to hold the electronic/computing components. The housing 212 (which is often referred to as the "can", "case", "encasing", or "case electrode") may be programmably selected to act as an electrode for certain sensing modes. The housing 212 may include a connector (not shown), such as a header, with at least one terminal and optionally additional terminals. In certain embodiments, the terminals may be coupled to electrodes 213 that are provided upon or immediately adjacent the housing 212. Optionally, more than two terminals may be provided in order to support more than two sensing electrodes, such as for a bipolar sensing scheme that uses the housing 212 as a reference electrode. Additionally or alternatively, the terminals may be connected to one or more leads each having one or more electrodes provided thereon, where the electrodes are located in various locations about the heart. The type and location of each electrode may vary.

The IMD 101 includes a programmable microcontroller 221 that controls various operations of the IMD 101, such as cardiac monitoring, and/or other types of physiologic monitoring. The microcontroller 221, which can be more generally referred to as a controller, can include a microprocessor (or equivalent control circuitry), random-access memory (RAM) and/or read only memory (ROM), logic and timing circuitry, state machine circuitry, and input/output (I/O) circuitry. The microcontroller 221 can also perform certain operations described herein in connection with collecting physiologic signal sample data, such as, but not limited to, EGM sample data.

A switch 217 is optionally provided to allow selection of different electrode configurations under the control of the microcontroller 221. The electrode configuration switch 217 may include multiple switches for connecting the desired electrodes 213 to the appropriate I/O circuits, thereby facilitating electrode programmability. The switch 217 is controlled by a control signal from the microcontroller 221. Optionally, the switch 217 may be omitted and the I/O circuits directly connected to the housing electrode and another electrode 213.

The microcontroller 221 can include an optional arrhythmia detector 234 that is configured to analyze cardiac activity signals to identify potential arrhythmia episodes (e.g., tachycardias, bradycardias, cardia pause, atrial fibrillation (AF), etc.). Although not shown, the microcontroller 221 may further include other dedicated circuitry and/or firmware/software components that assist in monitoring various conditions of the patient's heart and managing pacing therapies. The arrhythmia detector 234 of the microcontroller 221 can include an on-board arrhythmia detection process that detects arrhythmia episodes, such as AF episodes using R-R interval irregularities. The arrhythmia detector 234 may be implemented as firmware, software and/or circuits, including combinations thereof.

The IMD 101 is further equipped with a communication modem (modulator/demodulator) 240 to enable wireless communication. In one implementation, the communication modem 240 uses high frequency modulation, for example using Bluetooth^{®} or Bluetooth^{®} Low Energy (BLE) telemetry protocols. The signals are transmitted in a high frequency range and will travel through the body tissue in fluids without stimulating the heart or being felt by the patient. The communication modem 240 may be implemented in hardware as part of the microcontroller 221, or as software/firmware instructions programmed into and executed by the microcontroller 221. Alternatively, the communication modem 240 may reside separately from the microcontroller 221 as a standalone component. The communication modem 240 facilitates data retrieval from a remote monitoring network. The communication modem 240 enables timely and accurate data transfer directly from the patient to the ED utilized by a physician.

The IMD 101 includes a sensing circuit 224 coupled to, preferably selectively coupled to, one or more electrodes 213 that perform sensing operations, through the optional switch 217 to detect cardiac activity data indicative of cardiac activity. The sensing circuit 224 may include dedicated sense amplifiers, multiplexed amplifiers, or shared amplifiers. It may further employ one or more low power, precision amplifiers with programmable gain and/or automatic gain control, bandpass filtering, and threshold detection circuit to selectively sense the features of interest. In one embodiment, the switch 217 may be used to determine the sensing polarity of the cardiac signal by selectively closing the appropriate switches.

The output of the sensing circuit 224 is connected to the microcontroller 221 which, in turn, determines when to store EGM data for a segment of an EGM (digitized by an analog-to-digital (A/D) data acquisition system (DAS) 230) in the memory 250. For example, the microcontroller 221 may only store the EGM data (from the A/D data acquisition system 230) in the memory 250 when a potential arrhythmia episode is detected. The sensing circuit 224 may receive a control signal 226 from the microcontroller 221 for purposes of controlling the gain, threshold, polarization charge removal circuitry (not shown), and the timing of any blocking circuitry (not shown) coupled to the inputs of the sensing circuit.

Optionally, the IMD 101 may include multiple sensing circuits, similar to sensing circuit 224, where each sensing circuit is coupled to two or more electrodes and controlled by the microcontroller 221 to sense cardiac electrical activity detected at the corresponding two or more electrodes. The sensing circuit 224 may operate in a unipolar sensing configuration or in a bipolar sensing configuration. Optionally, the sensing circuit 224 may be removed entirely and the microcontroller 221 perform the operations described herein based upon the EGM from the A/D data acquisition system 230 directly coupled to the electrodes 213.

The IMD 101 further includes the above-mentioned A/D data acquisition system 230 coupled to one or more electrodes 213 optionally via the switch 217 to sample cardiac activity signals across any pair of desired electrodes. The A/D data acquisition system 230 is configured to acquire EGM signals (or segments thereof), convert the raw analog data into digital data, and store the digital data as EGM data for later processing and/or for real-time telemetric transmission to an ED 102 (e.g., a programmer, local transceiver, or a diagnostic system analyzer). The A/D data acquisition system 230 is preferably controlled by a control signal 236 from the microcontroller 221. The EGM may be utilized as the cardiac activity data that is analyzed for potential arrhythmia episodes. The arrhythmia detection algorithms may be applied to EGM from the sensing circuit 224 and/or the A/D data acquisition system 230.

The IMD 101 may further include magnet detection circuitry (not shown), coupled to the microcontroller 221, to detect when a magnet is placed over the IMD 101. A magnet may be used by a clinician to perform various test functions of the housing 212 and/or to signal the microcontroller 221 that the ED 102 is in place to receive or transmit data to the microcontroller 221 through the transceivers (TX/RX) 244. In accordance with an embodiment, the IMD 101 can start its advertising (e.g., BLE advertising) in response to the IMD 101 detecting that a magnet has been placed over the IMD 101.

The IMD 101 can optionally include one or more physiologic sensors 246. Signals generated by the physiological sensors 246 can be passed to the microcontroller 221 for analysis and optional storage in the memory 250 in connection with the cardiac activity data, markers, episode information and the like. While shown as being included within the housing 212, the physiologic sensor(s) 246 may be external to the housing 212, yet still be implanted within or carried by the patient. Examples of physiologic sensors might include sensors that, for example, sense temperature, respiration rate, pH of blood, ventricular gradient, activity, position/posture, minute ventilation (MV), cardiac electrical activity, cardiac mechanical activity, and so forth. Examples of such physiologic sensors 246 include accelerometers, pressure sensors, flow sensors, temperature sensors, and/or the like. Sample data of the physiologic signals produced by the physiologic sensor(s) 246 can be transmitted in real-time from the IMD 101 to the ED 102 so that the signal(s) can be displayed by the ED 102 on a display of (or communicatively coupled to) the ED 102.

The microcontroller 221 is coupled to a memory 250 preferably by a suitable data/address bus. The programmable operating parameters used by the microcontroller 221 are stored in memory 250 and used to customize the operation of the IMD 101 to suit the needs of a particular patient. Such operating parameters define, for example, detection rate thresholds, sensitivity, automatic features, arrhythmia detection criteria, activity sensing or other physiological sensors, and electrode polarity, etc.

In addition, the memory 250 stores the ECG and/or EGM data, as well as the markers and other data content associated with detection of arrhythmia episodes. The operating parameters of the IMD 101 may be non-invasively programmed into the memory 250 through a transceiver 244 in telemetric communication via the communication link 103 with the ED 102. The transceiver 244 is shown as being coupled to an antenna 205 that enables the IMD 101 to transmit and receive radio frequency (RF) signals, such as BLE signals, to and from the ED 102. The transceiver 244 also enables physiologic signal sample data (e.g., EGM sample data), classification data, etc. and status information relating to the operation of the IMD 101 (as contained in the microcontroller 221 or memory 250) to be sent to the ED 102 through the established communication link 103. In accordance with certain embodiments, the transceiver 244 is used to send real-time EGM sample data (and/or one or more other types of real-time physiologic signal sample data) to the ED 102, so that the ED 102 can display an EGM (and/or other type(s) of physiologic signal(s)) on a display of the ED 102 (or on a display that is communicatively coupled to the ED 102). In accordance with certain embodiments of the present technology described herein, the ED 102 can simultaneously display an ECG (and/or other type of further physiological signal) sensed by the ED 102, such that the two or more signals (e.g., the EGM and the ECG) that are being co-displayed are synchronized (aka in-sync) with one another.

The IMD 101 is also shown as including a real-time clock (RTC) 245, which can also be referred to as the IMD_RTC 245. In an embodiment, the IMD_RTC 245 monotonically counts up. In accordance with an embodiment, the IMD_RTC 245 reliably maintains and provides a current time through disruptive system states such as hangs, sleep, and reboots, without the need to have its time set again. The value of the IMD_RTC 245 can be provided to the microcontroller 221, and/or to the transceiver 244, and/or other components of the IMD 101.

A battery 248 provides operating power to all of the components in the IMD 101. The battery 248 is capable of operating at low current drains for long periods of time. The battery 248 also desirably has a predictable discharge characteristic so that elective replacement time can be detected. As one example, the IMD 101 employs lithium/silver vanadium oxide batteries. The battery 248 may afford various periods of longevity. In alternate embodiments, the battery 248 could be rechargeable.

### Example External Device (ED)

FIG. 3 illustrates example components of the example ED 102 for use in communicating with and/or programming the IMD 101. By way of example, the ED 102 may represent a bedside monitor installed in a patient's home and utilized to wirelessly communicate with the IMD 101 while the patient is at home, in bed or asleep. The ED 102 may be a programmer used in a clinic to interrogate the IMD 101, retrieve data and program detection criteria and other features. The ED 102 may be a handheld device (e.g., smartphone, tablet device, laptop computer, smartwatch and the like) that can be coupled over a network (e.g., the Internet) to a remote monitoring service, medical network and the like. The ED 102 can facilitate access by clinicians to patient data as well as permit the physician to review real-time EGM signals sensed by the IMD 101.

In certain embodiment, the ED 102 can be used to analyze EGM segments obtained and stored by the IMD 101. More generally, the ED 102 may permit a physician or other authorized user to program the operation of the IMD 101 and to retrieve and display data received from the IMD 101 such as EGM data and device diagnostic data. Further, the ED 102 may be capable of causing the IMD 101 to perform functions necessary to complete certain algorithms of the embodiments described herein. The ED 102 may also be capable of processing and analyzing data received from the IMD 101. Additionally, the ED 102 can be capable of accepting various user inputs. In accordance with certain embodiments, the ED 102 can be configured to provide for synchronous display, e.g., synchronous co-display, of a real-time signal sensed by the IMD 101 (and/or by another implantable device communicatively coupled to the IMD 101) and a real-time signal sensed by the ED 102 (and/or by another external device that is communicatively coupled to the ED 102).

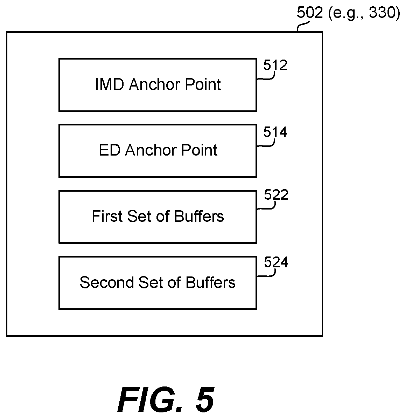

The ED 102 can be controlled by a controller 302, which may be a programmable microprocessor or microcontroller or may be a dedicated processing device such as an Application Specific Integrated Circuit (ASIC) or the like. In an embodiment, the controller 302 comprises a central processing unit (CPU). Software instructions to be performed by the controller 302 can be accessed via an internal bus 304 from a ROM 306 and RAM 330. Additional software may be accessed from a hard drive 308, floppy drive 310, and CD ROM drive 312, or other suitable permanent mass storage device. Depending upon the specific implementation, a Basic Input Output System (BIOS) is retrieved from the ROM 306 at power up. In accordance with certain embodiments of the present technology, the RAM 330 is used to store anchor points and to implement buffers that are used to implement specific embodiments, as explained in additional detail below with reference to FIG. 5.

Once operating, the controller 302 can display a menu of programming options to the user via a liquid-crystal display (LCD) display 314 or another suitable computer display device. To this end, the controller 302 may, for example, display a menu of specific programming parameters of the IMD 101 to be programmed or may display a menu of types of diagnostic data to be retrieved and displayed. The ED 102 can additionally or alternatively include and/or be communicatively coupled to various other types of displays on which physiologic signals can be displayed.

The ED 102 can include, or be communicatively coupled to, an ECG circuit 334 that is capable of sensing one or more ECG signals. The ECG circuit 334 can be coupled by cables to a plurality of skin electrode 335 that enable the ECG circuit 334 to sense one or more ECG signals. For one non-limiting example, the ECG circuit 334 can be coupled via cables (or wirelessly) to ten skin electrodes placed on a person's arms, legs and around their chest to sense the 12- or 14-channel ECG. The ten skin electrodes are named right arm (RA), left arm (LA), right leg (RL), left leg (LL), V1, V2, V3, V4, V5, V6 electrodes in the art. The V1, V2, V3, V4, V5, V6 electrodes are placed around the chest, and more specifically, the V1 electrode is place at the fourth intercostal space on the right sternum, the V2 electrode is placed at the fourth intercostal space at the left sternum, the V3 electrode is placed midway between placement of the V2 and V4 electrodes, the V4 electrode is placed at the fifth intercostal space at the midclavicular line, the V5 electrode is placed anterior an axillary line on the same horizontal level as the V4 electrode, and the V6 electrode is place mid-axillary line on the same horizontal level as the V4 and V5 electrodes. The limb electrodes RA, LA, RL, LL are placed on limbs, and more specifically, the RA electrode is placed anywhere between the right shoulder and right elbow, the RL electrode is placed anywhere below the right torso and above the right ankle, the LA electrode is placed anywhere between the left shoulder and the left elbow, and the LL electrode is placed anywhere below the left torso and above the left ankle. It would also be possible for the ECG circuit 334 to be coupled to more or less than ten skin electrodes.

Still referring to FIG. 3, the ED 102 is shown as including a telemetry subsystem 322. The telemetry subsystem 322 includes a transceiver 326 connected to an antenna 328 to enable the ED 102 to wirelessly communicate with the IMD 101 via a wireless communication protocol, such as BLE. The telemetry subsystem 322 can optionally include its own microcontroller 324 that is used to control communication between the ED 102 and the IMD 101. The ED 102 can additionally include a main controller (e.g., a CPU) 302 that controls other aspects of the ED 102. It would also be possible for the ED 102 to include only a single controller or more than two controllers. More generally, the ED 102 can include one or more controllers 302, 324 that controls the operations of the ED 102.

The ED 102 is also shown as including a real-time clock (RTC) 345, which can also be referred to as the ED_RTC 345. In an embodiment, the ED_RTC 345 monotonically counts up. In accordance with an embodiment, the ED_RTC 345 reliably maintains and provides a current time through disruptive system states such as hangs, sleep, and reboots, without the need to have its time set again. The value of the ED_RTC 345 can be provided to the controller 302, the controller 324, the telemetry subsystem 322, and/or other components of the ED 102.

The ED 102 can also include a Network Interface Card (NIC) 360 to permit transmission of data to and from other computer systems via a router 362 and Wide Area Network (WAN) 364. Alternatively, the ED 102 might include a modem for communication via the Public Switched Telephone Network (PSTN). Depending upon the implementation, the modem may be connected directly to internal bus 304 and may be connected to the internal bus 304 via either a parallel I/O port or circuit 340 or a serial I/O port or circuit 342. Data transmitted from other computer systems may include, for example, data regarding medication prescribed, administered, or sold to the patient.

The ED 102 can receive data from the IMD 101, including parameters representative of the current programming state of the IMD 101. The ED 102 can also receive EGM sample data (and/or sample data of one or more other types of physiologic signals) from the IMD 101. Any or all of the information displayed by ED 102 may also be printed using an optional printer 336.

An optional speaker 344 is included for providing audible tones to the user, such as a warning beep in the event improper input is provided by the physician. One or more peripheral devices may be connected to the ED 102 via the parallel I/O port or circuit 340 or the serial I/O port or circuit 342 as well. Although one of each is shown, a plurality of I/O ports or circuitries might be provided.

With the ED 102 configured as shown, a physician or other authorized user can retrieve, process, and display a wide range of information received from the IMD 101 and reprogram the IMD 101, including configurations of operating parameters, if needed. The descriptions provided herein with respect to FIG. 3 are intended merely to provide an overview of the operation of the example ED 102 and are not intended to describe in detail every feature of the hardware and software of the device and are not intended to provide an exhaustive list of the functions performed by the device.

### Example Method

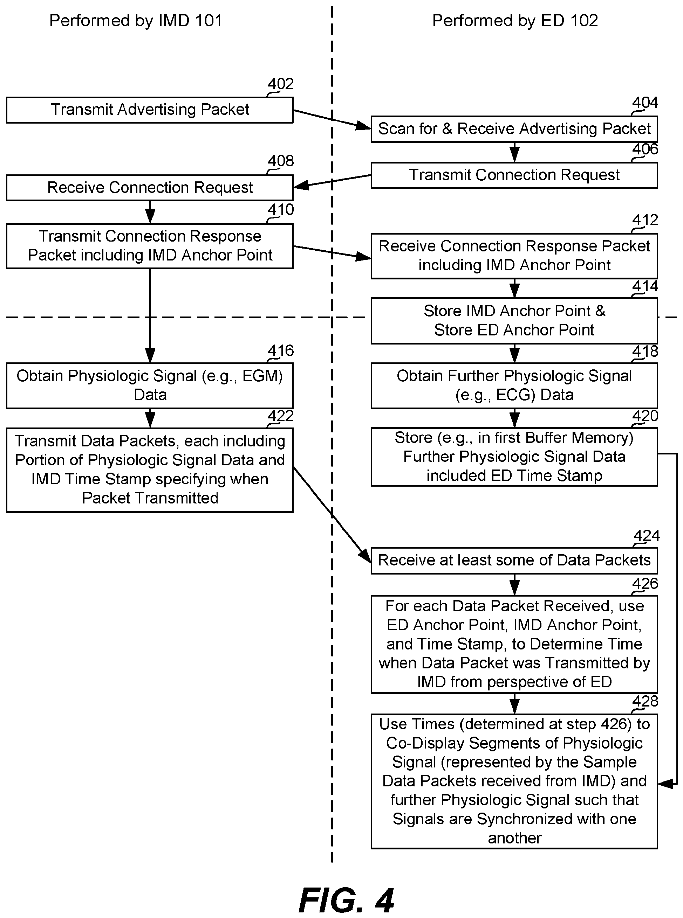

The high level flow diagram of FIG. 4 will now be used to describe a method according to an embodiment of the present technology for use with a system (e.g., system 100) including an ED (e.g., ED 102) and an IMD (e.g., IMD 101) configured to wirelessly communicate with one another, e.g., using BLE. As will be appreciated from the following discussion, such a method enables the ED to display (or more generally cause the display of) a plurality of different physiologic signals such that the different physiologic signals as co-displayed are synchronized with one another, even though the data for at least one of the physiologic signals is obtained by the IMD, and data for at least another one of the physiologic signals is obtained by the ED. In other words, the method enables the ED to compensate for data corresponding to the different physiologic signals traveling different communication paths and experiencing different signal processing delays, which if not compensated for, would result in the different signals when displayed being unsynchronized with one another. For example, the method can be used to co-display and EGM and an ECG such that they are synchronized with one another, where EGM data (used to display the EGM) is obtained by the IMD, and ECG data (used to display the ECG) is obtained by the ED.

In FIG. 4, the blocks or steps shown to the left of the vertical dashed lined are performed by the IMD (e.g., IMD 101), and the blocks or steps shown to the right of the vertical dashed line are performed by the ED (e.g., ED 102). The steps that are performed by the IMD can be performed by and/or under the control of one or more controller (e.g., microcontroller 221) of the IMD. For example, referring briefly back to FIG. 2, the steps that are performed by the IMD 101 can be performed by and/or under the control of the microcontroller 221. The steps that are performed by the ED can be performed by and/or under the control of one or more controller (e.g., main controller 302 and/or telemetry controller 324) of the ED 102. For example, referring briefly back to FIG. 3, the steps that are performed by the ED 102 can be performed by and/or under the control of the main controller 302 and/or the telemetry controller 324. While the IMD 101 in FIG. 2 was shown and described as having a single controller 221, it is possible that the IMD 101 can include multiple controllers. While the ED 102 in FIG. 3 was shown and described as including controller 302 and controller 324, it is possible that the ED 102 can include more than two controllers or a single controller.

Referring now to FIG. 4, step 402 involves the IMD transmitting one or more advertising packets in accordance with a wireless communication protocol, such as the BLE protocol. In certain embodiments, the IMD begins to transmit the advertising packets in response to the IMD detecting that a magnet has been placed in close proximity to the IMD. The IMD can alternatively, or additionally, begin to transmit the advertising packets in response to one or more other types of triggering events, such as the IMD not having communicated with an ED for at least a specified amount of time, or in response to the IMD detecting an alert or other specific condition, but not limited thereto. In certain embodiments, the IMD can additionally or alternatively transmit advertising packets in accordance with one or more predetermined schedules. In an embodiment, the IMD transmits the advertising packets on three BLE channels, e.g., channel 37 (2402 MHz), channel 38 (2426 MHz), and channel 39 (2480 MHz) for each advertising event. The advertising packets may be sent at an advertising interval which specifies the time between advertising events.

Still referring to FIG. 4, step 404 involves the ED scanning for and receiving the advertising packet. In response to the ED receiving the advertising packet from the IMD, the ED transmits a connection request to the IMD at step 406. The connection request transmitted by the ED may include connection parameters for establishing a wireless connection, such as a BLE connection, where the parameters may, for instance, include a connection interval indicating how often the IMD and the ED will communicate and a channel map identifying the channels on which they will communicate. The IMD may accept the connection request by tuning into the right frequency at the right time to send a connection response and establish the wireless connection, e.g., the BLE connection. Reference to establishing a wireless connection between the IMD and ED as referred to herein may be implemented by establishing a wireless connection, such as the BLE connection, between the transceiver of the IMD (e.g., the transceiver 244 in FIG. 2) and the transceiver of the ED (e.g., the transceiver 326 in FIG. 3).

Step 408 involves the IMD receiving the connection request packet, within a receive window following the IMD transmitting the advertising packet. In accordance with an embodiment, in response to receiving the connection request packet, at step 410 the IMD transmits a connection response packet including an IMD anchor point. In accordance with an embodiment, the IMD anchor point is a value of a real-time clock (RTC) of the IMD (e.g., the IMD_RTC 245 of FIG. 2) when the IMD transmits the connection response packet that is used to establish a wireless connection between the IMD and the ED. As will be appreciated from the below description, the ED will use the IMD anchor point to display (or more generally cause the display of) the physiologic signal (for which data is obtained at step 416) such that the displayed physiologic signal is synchronized with one or more other physiologic signals for which data is obtained by the ED.

Step 412 involves the ED receiving the connection response packet including the IMD anchor point, which was transmitted at step 410. At step 414, in response to receiving the connection response packet including the IMD anchor point, the ED stores, in memory (e.g., RAM 330 of FIG. 3), the IMD anchor point (received from the IMD at step 412), and the ED also stores, in memory (e.g., RAM 330 of FIG. 3) an ED anchor point. In accordance with an embodiment, the ED anchor point is a value of a real-time clock (RTC) of the ED (e.g., the ED_RTC 345 in FIG. 3) when the ED receives the connection response packet from the IMD. Referring briefly back to FIG. 3, the memory in which the ED stores the IMD anchor point (received from the IMD at step 412), and ED anchor point, can be a portion of the RAM 330, but is not limited thereto.

The IMD anchor point and the ED anchor point correspond to when a wireless connection (e.g., a BLE connection) is established between the ED and the IMD. In other words, the IMD anchor point and the ED anchor point are indicative of a point in time when the wireless connection is established between the ED and IMD. Accordingly, it could be appreciated that once the IMD anchor point and the ED anchor point are stored by the ED, a wireless connection (e.g., a BLE connection) is established between the ED and the IMD. Once the wireless connection (e.g., the BLE connection) is established, the IMD and the ED can communicate with one another on/at the identified channels and times. BLE, for example, has specified data channels 0-36 that may be used for BLE communication sessions. Referring again to FIG. 4, the blocks or steps in FIG. 4 that are shown above the horizontal dashed line occur prior to the wireless connection (e.g., the BLE connection) being established between the ED and the IMD, and the blocks or steps shown below the horizontal dashed line occur after and while the wireless connection (e.g., the BLE connection) is established between the ED and the IMD.

Step 416 involves the IMD obtaining physiologic signal data for a patient within which the IMD is implanted. For example, the IMD can sense an EGM using its electrodes and can sample the sensed EGM using an analog-to-digital converter (ADC) or A/D data acquisition system (e.g., the A/D data acquisition system 230 in FIG. 2), in which case the physiologic signal data can be referred to as EGM data. For another example, the IMD can sense a pressure signal using a pressure sensor of the IMD (or communicatively coupled to the IMD) (e.g., the physiologic sensor 246 in FIG. 2) and can sample the pressure signal using an ADC, in which case the physiologic signal data can be referred to as pressure signal data. More generally, the term physiologic signal, as used to herein, refers to an analog or digital electrical signal sensed using two or more electrodes (e.g., electrodes 213 in FIG. 2) or a sensor, and optionally processed, e.g., using one or more amplifiers and/or one or filters, but not limited thereto. Examples of physiologic signals include, but are not limited to, an EGM, a subcutaneous ECG signal, a pressure signal, a cardiac impedance signal, a respiratory signal, a PPG signal, an IPG signal, a temperature signal, and/or the like. The physiologic signal data that is obtained by the IMD at step 416 can correspond to any of these types of physiologic signals, but is not limited thereto.

Step 422 involves the IMD transmitting a plurality of data packets to the ED, wherein each data packet, of the plurality of data packets that are transmitted by the IMD to the ED, includes a portion of the physiologic signal data (obtained by the IMD) and a respective IMD time stamp specifying when the data packet is transmitted by the IMD. In accordance with an embodiment, the IMD time stamp is a value of the RTC of the IMD (aka the IMD_RTC) when the IMD transmits the data packet to the ED. The portion of the physiologic signal data included in each of the data packets can correspond to a different segment of a specified duration (e.g., 500 msec, but not limited thereto) of the physiologic signal. For example, each EGM data packet can include EGM data corresponding to 500 msec of an EGM signal that was sensed by the IMD (or by another implantable device that is communicatively coupled to the IMD).

Step 418 involves the ED obtaining further physiologic signal data for the patient within which the IMD is implanted. For example, the ED can sense an ECG using skin electrodes and can sample the sensed ECG using an ADC (e.g., ECG circuit 334 in FIG. 3), in which case the further physiologic signal data can be referred to as ECG data. For another example, the ED can sense a PPG signal using a PPG sensor placed on a patient's finger, and can sample the PPG signal using an ADC, in which case the further physiologic signal data can be referred to as PPG signal data (or more succinctly as PPG data). For still another example, the ED can sense a blood pressure signal using a pressure cuff, or the like, and can sample the blood pressure signal using an ADC, in which case the further physiologic signal data can be referred to as blood pressure signal data (or more succinctly as blood pressure data). More generally, the ED can obtain its further physiologic signal using two or more electrodes (e.g., electrodes 335 in FIG. 3) or a sensor of the ED or communicatively coupled to the ED. The above examples of the further physiologic signal for which the ED can obtain data are not intended to be all encompassing. Such signals can be processed, including but not limited to amplified and/or filtered, before and/or after being sampled, as is known in the art.

Step 420 involves the ED storing, preferably in buffer memory, portions (e.g., packets) of the further physiologic data along with ED time stamps specifying, for each of the portions (e.g., packets) of the further physiologic data, when the portion (e.g., packet) of the further physiologic data was stored. In accordance with an embodiment, the ED time stamp is a value of the RTC of the ED (aka the ED_RTC) when the ED stores the portion (e.g., packet) of the further physiologic data. Referring briefly back to FIG. 3, the buffer memory that is used to store portions (e.g., packets) of the further physiologic data along with ED time stamps, can be implemented by a portion of the RAM 330, but is not limited thereto.

The performance of steps 416 and 422 by the IMD overlaps in time with the performance of steps 418 and 420 by the ED. For example, the IMD can obtain EGM data (at step 416) and transmit EGM data packets along with IMD time stamps (at step 418) while at the same time the ED obtains ECG data (at step 418) and stores ECG data along with ED time stamps (at step 420).

Still referring to FIG. 4, step 424 involves the ED receiving at least some of the data packets that were transmitted by the IMD (at step 422). Preferably the ED receives all of the data packets that were transmitted by the IMD (at step 422). However, due to noise and/or other types of interference, it is possible that one or more of the data packets that are transmitted by the IMD are dropped or otherwise not received by the ED.

Step 426 involves the ED determining, for each data packet received by the ED (at step 422), a respective time when the data packet was transmitted by the IMD from a perspective of the ED, based on the ED anchor point that was stored by the ED in memory, the IMD anchor point that was stored by the ED in memory, and the IMD time stamp included in the data packet received by the ED.

In accordance with an embodiment, at step 426 the ED determines the respective time when a data packet was transmitted by the IMD from the perspective of the ED, for each data packet received by the ED, using the following equation:${RT}_{DATA} = {ANCHOR}_{ED} + {TIME_STAMP}_{IMD} - {ANCHOR}_{IMD} ,$ where
RT_{DATA} is the respective time when the data packet was transmitted by the IMD from the perspective of the ED, which respective time is determined by the ED in step 426,
ANCHOR_{ED} is the ED anchor point stored by the ED in memory,
TIME_STAMP_{IMD} is the IMD time stamp included in the data packet received by the ED, and
ANCHORI_{MD} is the IMD anchor point stored by the ED in memory.

Still referring to FIG. 4, step 428 involves the ED using the respective times (e.g., RT_{DATA}) determined for the data packets received by the ED from the IMD, to cause displaying of respective segments of a physiologic signal that are represented by the data packets. Additionally, step 428 involves the ED causing display of the further physiologic signal, based on the further physiologic signal data and its respective time stamps, such that both signals as co-displayed are synchronized with one another. More specifically, in accordance with an embodiment, the ED controls one or more buffers of the ED to cause the further physiologic signal and the co-displayed physiologic signal to be synchronized with one another. For example, one type of physiologic signal data (e.g., EGM data) can be stored in a first set of buffers (which includes a least one buffer), while another type of physiologic signal data (e.g., ECG data) can be stored in a second set of buffers (which includes at least one further buffer), and a controller (e.g., controller 302 in FIG. 3) of the ED can control how the data is output from the buffers (e.g., buffers in RAM 330 in FIG. 3) and used to display corresponding physiologic signals (e.g., an EGM and an ECG) such that the multiple physiologic signals are synchronized with one another. Continuing with this example, an EGM and an ECG that are co-displayed are synchronized with one another where features (e.g., R-waves, P-waves, T-waves, etc.) of the two different signals that correspond to a same cardiac event are temporally aligned with one another. Referring briefly back to FIG. 3, the first sets of buffers can be implemented by a first portion of the RAM 330, and the second set of buffers can be implemented by a second portion of the RAM 330. Still referring briefly back to FIG. 3, the physiologic signals that are displayed such that they are synchronized with one another can be co-displayed on the LCD display 314, a touch screen 314, or can be displayed on a printout produced by the printer 336, but are not limited thereto. Other variations are also possible and within the scope of the embodiments described herein.

In accordance with an embodiment, the physiologic signal data, that is obtained by the IMD at step 416 is determined based on a real-time physiologic signal that is sensed by the IMD or by a further implantable device that is communicatively coupled to the IMD. Further, the physiologic signal data, that is obtained by the IMD, comprises real-time physiologic signal data. In an embodiment, the plurality of data packets, that are transmitted by the IMD to the ED, comprise real-time data packets. As the term is used herein, a real-time data packet is a data packet (corresponding to a segment of a physiologic signal) that is generated and transmitted from the IMD to the ED within 1 second of the segment of the signal being sensed. In accordance with an embodiment, the physiologic signals that are caused to be displayed at step 428 are real-time or near-real-time physiologic signals. As the term is used herein, a real-time physiologic signal is one that is displayed within 1 second of being sensed (i.e., the temporal offset between the actual sensed signal and the displayed signal is less than 1 second) preferably within 750 millisecond (ms) of being sensed, more preferably within 500 ms of being sensed, and most preferably within 250 ms of being sensed. As the term is used herein, a near- real-time physiologic signal is one that is displayed within between 1 second and 5 seconds of being sensed, i.e., the temporal offset between the actual sensed signal and the displayed signal is within the range of 1 to 5 seconds.

FIG. 5 illustrates an example memory 502 of the ED 102, or at least accessible by the ED 102, including a memory portion 512 that stores the IMD anchor point, and a memory portion 514 that stores the ED anchor point. The memory 502 is also shown as including a first set of buffers 522 used to store one type of physiologic signal data (e.g., EGM data) received from the IMD using the wireless connection (e.g., BLE connection), and a second set of buffers 524 used to store another type of physiologic signal data (e.g., ECG data) sensed by the ED 102 or by another non-implanted device communicatively coupled to the ED 102. The memory 502 can be implemented by the RAM 330 in FIG. 3, but is not limited thereto. A controller of the ED 102, such as the main controller 302 and/or telemetry controller 324 in FIG. 3, but not limited thereto, can be used to control the storage of the anchor points in the memory portions 512 and 514, respectively. Such controller(s) can also be used to control how and when signal data is output from the sets of buffers 522, 524 and used to co-display the physiologic signals (for which the signal data is stored in the sets of buffers 522, 524) such that the signals are synchronized with one another.

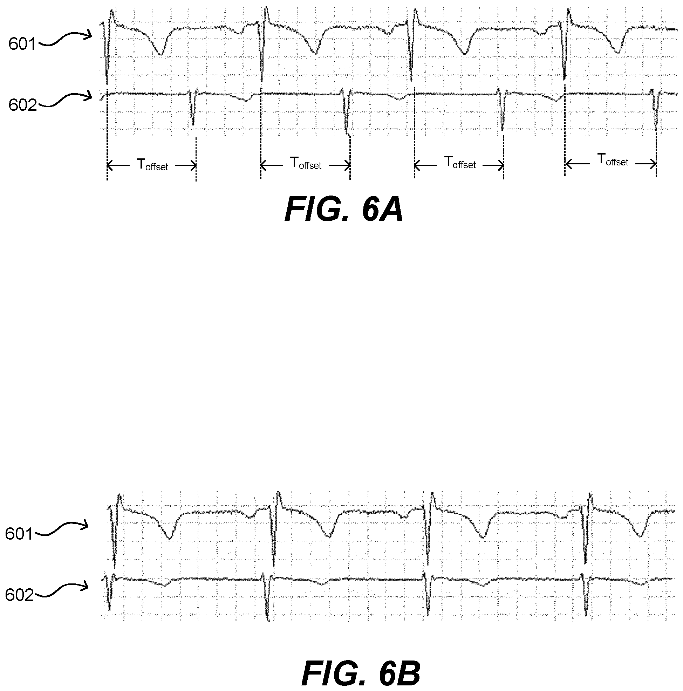

FIG. 6A shows examples of an EGM 601 and an ECG 602 displayed under the control of an ED (e.g., ED 102), where the EGM 601 is displayed based on EGM data obtained by an IMD (e.g., IMD 101) using implanted electrodes and transmitted to the ED using a wireless communication protocol, such as BLE, and where the ECG 602 is displayed based on ECG data obtained by the ED using non-implanted skin electrodes. The peaks of largest amplitudes in the EGM 601 and the ECG 602 in FIG. 6A corresponding to intrinsic ventricular depolarizations. Explained another way, peaks of largest amplitudes in the EGM 601 and the ECG 602 in FIG. 6A correspond to R-waves. As can be appreciated from FIG. 6A, the R-waves in the EGM 601 are shown as being significantly temporally offset from the R-waves in the ECG 602. The significant temporal offset between the displayed EGM 601 and the displayed ECG 602 in FIG. 6A can be due to the ED not properly compensating for the different paths that the ECG data and the EGM data may travel before being provided for display by the ED. The significant temporal offset between the displayed EGM 601 and the displayed ECG 602 in FIG. 6A can additionally be due to the ED not properly compensating for the EGM data being transmitted in a noisy environment where the wireless connection (e.g., the BLE connection) between the IMD and the ED is relatively poor. The significant temporal offset between the displayed EGM 601 and the displayed ECG 602 in FIG. 6A can additionally be due to the ED not properly compensating for the EGM data being transmitted by the IMD using a wireless communication protocol (e.g., BLE) that transmits data in short bursts rather than in a constant stream.

FIG. 6B is similar to FIG. 6A, in that FIG. 6B shows that same EGM 601 and the same ECG 602 that were shown in FIG. 6A. However, in FIG. 6B the ED has used an embodiment of the present technology to properly compensate for the different paths that the ECG data and the EGM data may travel before being provided for display by the ED, as well as to compensate for the EGM data being transmitted in a noisy environment and the EGM data being transmitted in short bursts rather than in a constat stream. As can be appreciated from FIG. 6B, the R-waves in the EGM 601 are shown as being temporally aligned with the R-waves in the ECG 602.

For much of the above discussion, the ED was described as causing the display of two physiologic signals such that they are synchronized with one another, where one of the physiologic signals is displayed based on data obtained from the IMD using implanted electrodes or an implanted sensor and transmitted to the IMD, e.g., using BLE, and where the other one of the physiologic signals is displayed based on data obtained by the ED using non-implanted electrodes or a non-implanted sensor. Examples of such displayed physiologic signals include the EGM 601 and the ECG 602 shown in FIG. 6B. However, it should be understood that embodiments of the present technology can also be used by the ED to display other types of physiologic signals that are displayed based on physiologic signal data obtained from the IMD (using one or more implanted electrodes and/or one or more implanted sensors) and/or based on physiologic signal data obtained by the ED (using one or more non-implanted electrodes and/or non-implanted sensors), such that all the displayed physiologic signals are displayed synchronized (in-sync) with one another.

In the above description, the first and second physiologic signals that were described as being sensed and co-displayed such that they were synchronized with one another were often described as being an EGM (or sub-ECG) sensed by an IMD and an ECG sensed by an ED (or by external electrodes communicatively coupled to the ED). However, it should be understood that embodiments of the present technology described herein are not limited to use with these specific types of physiologic signals. Examples of other types of first and second physiologic signals that may be sensed and can correspond to the first or second physiologic signals being displayed include, but are not limited to, a pressure signal, a cardiac impedance signal, a respiratory signal, a photoplethysmography (PPG) signal, an impedance plethysmography (IPG) signal, a temperature signal, a flow signal, just to name a few. For example, the first physiologic signal can be an EGM and or IPG signal sensed by an IMD, and the second physiologic signal can be a PPG signal sensed using a PPG sensor paced on finger, earlobe, or forehead, but not limited thereto. For another example, the first physiologic signal can be an arterial pressure signal sensed by an IMD (or an implanted sensor communicative coupled to the IMD) and the second physiologic signal can be a respiratory signal sensed by an external respirator sensor of an ED or communicatively coupled to the ED. These are just a few examples which are not intended to be all encompassing.

While embodiments of the present technology described above were often described as being used where the wireless communication between an IMD and an ED is implemented using BLE, this invention can also be used with other types of radio frequency (RF) communication besides BLE, such as with classic Bluetooth^{®}, ZigBee^{®}, Wireless Universal Serial Bus (USB), and Medical Implant Communication Service (MISC), but not limited thereto. It is also possible that embodiments of the present technology can be used with other types of wireless communication besides RF communication, such as inductive communication or conductive communication. However, where the wireless communication between an IMD and an ED is performed using inductive communication or conductive communication, the problems being overcome using an embodiment of the present technology may not be present, in which case there may be no need to utilize an embodiment of the present technology. Nevertheless, it should be understood that embodiments of the present technology can be used whenever there is a desire to improve the synchronization of signals being displayed, wherein one of the signals is obtained by an IMD and the other one of the signals is obtained by an ED, and wherein the IMD and the ED communicate with one another using wireless communication.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, it is noted that the term "based on" as used herein, unless stated otherwise, should be interpreted as meaning based at least in part on, meaning there can be one or more additional factors upon which a decision or the like is made. For example, if a decision is based on the results of a comparison, that decision can also be based on one or more other factors in addition to being based on results of the comparison.

Embodiments have been described above with the aid of functional building blocks illustrating the performance of specified functions and relationships thereof. The boundaries of these functional building blocks have often been defined herein for the convenience of the description. Alternate boundaries can be defined so long as the specified functions and relationships thereof are appropriately performed. Any such alternate boundaries are thus within the scope of the claimed invention. For example, it would be possible to combine or separate some of the steps shown in the various flow diagrams. It would also be possible to just perform a subset of the steps shown in the various flow diagrams. For another example, it is possible to change the boundaries of some of the block diagrams.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the embodiments without departing from its scope. While the dimensions, types of materials and coatings described herein are intended to define the parameters of the embodiments of the present technology, they are by no means limiting and are exemplary embodiments. Many other embodiments will be apparent to those of skill in the art upon reviewing the above description. The scope of the embodiments of the present technology should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. An external device, ED, (102) configured to wirelessly communicate with an implantable medical device, IMD, (101) the ED (102) comprising:
a memory (330);
a transceiver (326) that enables the ED (102) to wirelessly communicate with the IMD (101); and
at least one controller (302, 324) coupled to the transceiver (326) and the memory (330);
wherein while a wireless connection is in a process of being established between the ED (102) and the IMD (101), the at least one controller (302, 324) is/are configured to:
control the transceiver (326) to transmit a connection request packet to the IMD (101) in response to receiving an advertising packet from the IMD (101) to thereby enable the wireless connection to be established between the ED (102) and the IMD (101);
control the transceiver (326) to receive from the IMD (101) an IMD anchor point transmitted by the IMD (101) in response to the IMD (101) receiving the connection request packet from the ED (102);
save in the memory (330) the IMD anchor point; and
determine an ED anchor point and also save in the memory (330) the ED anchor point; and
wherein while the wireless connection is established between the ED (102) and the IMD (101), the at least one controller (302, 324) is/are configured to:
control the transceiver (326) to receive at least some of a plurality of data packets from the IMD (101), wherein each data packet of the plurality of data packets that are received from the IMD (101) includes a portion of physiologic signal data and a respective time stamp included by the IMD (101);
determine, for each said data packet of the data packets received from the IMD (101), a respective time when the data packet was transmitted by the IMD (101) from a perspective of the ED (102) based on the ED anchor point that is stored in the memory (330), the IMD anchor point that is stored in the memory (330), and the respective time stamp included in the data packet received from the IMD (101);
obtain further physiologic signal data representative of a further physiologic signal sensed using one or more skin electrodes (335) or a non-implanted sensor; and
use the respective times, determined for the data packets received from the IMD (101), to cause co-displaying of respective segments of a physiologic signal that are represented by the data packets along with the further physiologic signal such that they are synchronized with one another on a display (314, 316) of or communicatively coupled to the ED (102).

2. The ED (102) of claim 1, wherein the IMD anchor point and the ED anchor point correspond to when a wireless connection is established between the ED (102) and the IMD (101).

3. The ED (102) of any one of claims 1 or 2, wherein the at least one controller (302, 324) is/are configured to determine the respective time when a said data packet was transmitted by the IMD (101) from a perspective of the ED (102), for each said data packet of the data packets received by the ED (102), using an equation as follows:${RT}_{DATA} = {ANCHOR}_{ED} + {TIME_STAMP}_{IMD} - {ANCHOR}_{IMD} ,$ where
RT_{DATA} is the respective time when the data packet was transmitted by the IMD (101) from the perspective of the ED (102), which respective time is determined by the ED (102),
ANCHOR_{ED} is the ED anchor point stored by the ED (102),
TIME_STAMP_{IMD} is the IMD time stamp included in the data packet received by the ED (102), and
ANCHOR_{IMD} is the IMD anchor point stored by the ED (102).

4. The ED (102) of any one of claims 1 through 3, further comprising:
one or more buffers (330); and
wherein the at least one controller (302, 324) is/are further configured to control the one or more buffers (330) to cause the further physiologic signal and the physiologic signal be synchronized with one another when they are co-displayed on the display (314, 316) of or communicatively coupled to the ED (102).

5. The ED (102) of any one of claims 1 through 4, wherein:
the physiologic signal comprises one of an electrogram, EGM, or a subcutaneous electrocardiogram, ECG; and
the further physiologic signal, that is co-displayed and synchronized with the one of the EGM or the subcutaneous ECG, comprises an electrocardiogram, ECG, sensed using the one or more skin electrodes (335).

6. A system (100) comprising the ED (102) of any one of claims 1 through 5, and further comprising the IMD (101), wherein the IMD (101) comprises:
a memory (250) of the IMD (101);
a transceiver (244) of the IMD (101) that enables the IMD (101) to wirelessly communicate with the ED (102); and
at least one controller (221) of the IMD (101) coupled to the transceiver (244) of the IMD (101) and the memory (250) of the IMD (101);
wherein while the wireless connection is established between the transceiver (326) of the ED (102) and the transceiver (244) of the IMD (101), the at least one controller (221) of the IMD (101) is/are configured to:
obtain the physiologic signal data representative of the physiologic signal for the patient within which the IMD (101) is implanted; and
control the transceiver (244) of the IMD (101) to transmit the plurality of data packets to the ED (102), wherein each data packet, of the plurality of data packets that are transmitted by the IMD (101) to the ED (102), includes a portion of the physiologic signal data and a respective time stamp specifying when the data packet is transmitted by the IMD (101) from a perspective of the IMD (101).

7. The system (100) of claim 6, wherein while the wireless connection is in the process of being established between the transceiver (326) of the ED (102) and the transceiver (244) of the IMD (101), the at least one controller (221) of the IMD (101) is/are configured to control the transceiver (244) of the IMD (101) to transmit an advertising packet, receive the connection request packet from the ED (102) within a receive window following when the IMD (101) transmits the advertising packet, and in response thereto, transmit to the ED (102) a connection response packet including the IMD anchor point.

8. The system of any one of claims 6 or 7, wherein:
the IMD (101) includes a real-time clock, RTC (245);
the IMD anchor point comprises a value of the RTC (245) of the IMD (101) when a connection response packet is transmitted by the IMD (101) in response to the IMD (101) receiving the connection request packet from the ED (102); and
the respective time stamp of each said data packet of the data packets received by the ED (102) from the IMD (101) comprises a respective value of the RTC (245) of the IMD (101) when the data packet is transmitted by the IMD (101).

9. The system of claim 8, wherein:
the ED (102) includes a real-time clock, RTC (345); and
the ED anchor point comprises a value of the RTC (345) of the ED (102) when the ED (102) receives, from the IMD (101), the connection response packet.

10. The system of any one of claims 6 through 9, wherein:
the physiologic signal data, that is obtained by the IMD (101), is determined based on a real-time physiologic signal that is sensed by the IMD (101) or by a further implantable device communicatively coupled to the IMD (101);
the further physiologic signal data, that is obtained by the ED (102), is determined based on a further real-time physiologic signal that is sensed by the ED (102) or by a further external device communicatively coupled to the ED (102); and
the respective segments of the physiologic signal and the further physiologic signals are co-displayed in real-time or near-real-time such that they are synchronized with one another.

11. A method for synchronous display of signal segments, the method comprising: an external device, ED (102), transmitting a connection request packet to an implantable medical device, IMD, (101) in response to receiving an advertising packet from the IMD (101) to thereby enable establishment of a wireless connection between the ED (102) and the IMD (101);
the ED (102) receiving, during a process of establishing the wireless connection between the ED (102) and the IMD (101), an IMD anchor point transmitted by the IMD (101) in response to the IMD (101) receiving the connection request packet;
the ED (102) storing the IMD anchor point and an ED anchor point;
the ED (102) receiving, while the wireless connection is established between the ED (102) and the IMD (101), a plurality of data packets, each of which includes a portion of physiological signal data for a patient obtained by the IMD (101) and a respective IMD time stamp specifying when the data packet is transmitted by the IMD (101);
the ED (102) determining, for each said data packet received by the ED (102), a respective time when the data packet was transmitted by the IMD (101) from a perspective of the ED (102) based on the ED anchor point that is stored by the ED (102), the IMD anchor point that is stored by the ED (102), and the IMD time stamp included in the data packet received by the ED (102);
the ED (102) obtaining further physiologic signal data representative of a further physiologic signal sensed using one or more skin electrodes or a non-implanted sensor; and
the ED (102) using the respective times, determined for the data packets received by the ED (102) from the IMD (101), to cause co-displaying of respective segments of a physiologic signal that are represented by the data packets along with the further physiologic signal such that they are synchronized with one another.

12. The method of claim 11, wherein the ED (102) determining the respective time when a said data packet was transmitted by the IMD (101) from the perspective of the ED (102), is performed by the ED (102) for each said data packet of the data packets received by the ED (102), using an equation as follows:${RT}_{DATA} = {ANCHOR}_{ED} + {TIME_STAMP}_{IMD} - {ANCHOR}_{IMD} ,$ where
RT_{DATA} is the respective time when the data packet was transmitted by the IMD (101) from the perspective of the ED (102), which respective time is determined by the ED (102),
ANCHOR_{ED} is the ED anchor point stored by the ED (102),
TIME_STAMP_{IMD} is the IMD time stamp included in the data packet received by the ED (102), and
ANCHOR_{IMD} is the IMD anchor point stored by the ED (102).

13. The method of any one of claims 11 or 12, wherein:
the IMD (101) and the ED (102) each includes a respective real-time clock, RTC (245, 345);
the IMD anchor point received by the ED (102) comprises a value of the RTC (245) of the IMD (101) when a connection response packet is transmitted by the IMD (101) in response to the IMD (101) receiving the connection request packet from the ED (102); and
the respective time stamp of each said data packet of the data packets received by the ED (102) from the IMD (101) comprises a value of the RTC (245) of the IMD (101) when the data packet is transmitted by the IMD (101).

14. The method of claim 13, wherein:
the ED anchor point comprises a value of the RTC (345) of the ED (102) when the ED receives, from the IMD (101), the connection response packet.

15. The method of any one of claims 11 through 14, wherein:
the physiologic signal data, that is obtained by the IMD (101), is determined based on a real-time physiologic signal that is sensed by the IMD (101) or by a further implantable device communicatively coupled to the IMD (101);
the further physiologic signal data, that is obtained by the ED (102), is determined based on a further real-time physiologic signal that is sensed by the ED (102) or by a further external device communicatively coupled to the ED (102); and
the respective segments of the physiologic signal and the further physiologic signals are co-displayed in real-time or near-real-time such that they are synchronized with one another.

## Patentansprüche

1. Externe Vorrichtung, ED, (102), die dazu konfiguriert ist, drahtlos mit einer implantierbaren medizinischen Vorrichtung, IMD, (101) zu kommunizieren, wobei die ED (102) umfasst:
einen Speicher (330);
einen Sendeempfänger (326), der es der ED (102) ermöglicht, drahtlos mit der IMD (101) zu kommunizieren; und
mindestens eine Steuerung (302, 324), die mit dem Sendeempfänger (326) und dem Speicher (330) gekoppelt ist;
wobei, während sich eine drahtlose Verbindung zwischen der ED (102) und der IMD (101) im Herstellungsprozess befindet, die mindestens eine Steuerung (302, 324) dazu konfiguriert ist:
Steuern des Sendeempfängers (326), um als Reaktion auf Empfangen eines Advertising-Pakets von der IMD (101) ein Verbindungsanforderungspaket an die IMD (101) zu übertragen, um dadurch zu ermöglichen, dass die drahtlose Verbindung zwischen der ED (102) und der IMD (101) hergestellt wird;
Steuern des Sendeempfängers (326), um von der IMD (101) einen IMD-Ankerpunkt zu empfangen, der durch die IMD (101) als Reaktion darauf übertragen wird, dass die IMD (101) das Verbindungsanforderungspaket von der ED (102) empfängt;
Speichern des IMD-Ankerpunkts in dem Speicher (330); und
Bestimmen eines ED-Ankerpunkts und ebenfalls Speichern des ED-Ankerpunkts in dem Speicher (330); und
wobei, während die drahtlose Verbindung zwischen der ED (102) und der IMD (101) hergestellt wird, die mindestens eine Steuerung (302, 324) dazu konfiguriert ist:
Steuern des Sendeempfängers (326), um mindestens einige einer Mehrzahl von Datenpaketen von der IMD (101) zu empfangen, wobei jedes Datenpaket der Mehrzahl von Datenpaketen, die von der IMD (101) empfangen wird, einen Abschnitt physiologischer Signaldaten und einen jeweiligen Zeitstempel umfasst, den die IMD (101) beinhaltet;
Bestimmen einer jeweiligen Zeit, zu der das Datenpaket durch die IMD (101) aus einer Perspektive der ED (102) übertragen wurde, für jedes Datenpaket der Datenpakete, die von der IMD (101) empfangen werden, basierend auf dem ED-Ankerpunkt, der in dem Speicher (330) gespeichert ist, dem IMD-Ankerpunkt, der in dem Speicher (330) gespeichert ist, und dem jeweiligen Zeitstempel, der in dem Datenpaket umfasst ist, das von der IMD (101) empfangen wird;
Erhalten weiterer physiologischer Signaldaten, die ein weiteres physiologisches Signal darstellen, das unter Verwendung einer oder mehrerer Hautelektroden (335) oder eines nicht implantierten Sensors erfasst wird; und
Verwenden der jeweiligen Zeiten, die für die von der IMD (101) empfangenen Datenpakete bestimmt sind, um ein gemeinsames Anzeigen jeweiliger Segmente eines physiologischen Signals zu veranlassen, die durch die Datenpakete dargestellt sind, zusammen mit dem weiteren physiologischen Signal, sodass sie auf einer Anzeige (314, 316), die die ED (102) aufweist oder die damit kommunikativ gekoppelt ist, miteinander synchronisiert werden.

2. ED (102) nach Anspruch 1, wobei der IMD-Ankerpunkt und der ED-Ankerpunkt dem entsprechen, wenn eine drahtlose Verbindung zwischen der ED (102) und der IMD (101) hergestellt ist.

3. ED (102) nach einem der Ansprüche 1 oder 2, wobei die mindestens eine Steuerung (302, 324) dazu konfiguriert ist, die jeweilige Zeit, zu der ein das Datenpaket durch die IMD (101) aus einer Perspektive der ED (102) übertragen wurde, für jedes Datenpaket der Datenpakete, die durch die ED (102) empfangen werden, unter Verwendung einer folgenden Gleichung zu bestimmen:${RT}_{DATA} = {ANCHOR}_{ED} + {TIME_STAMP}_{IMD} - {ANCHOR}_{IMD} ,$ wobei
RT_{DATA} die jeweilige Zeit ist, zu der das Datenpaket durch die IMD (101) aus der Perspektive der ED (102) übertragen wurde, wobei die jeweilige Zeit durch die ED (102) bestimmt wird,
ANCHOR_{ED} der ED-Ankerpunkt ist, der durch die ED (102) gespeichert ist,
TIME_STAMP_{IMD} der IMD-Zeitstempel ist, der in dem Datenpaket umfasst ist, das von der ED (102) empfangen wird, und
ANCHOR_{IMD} der durch die ED (102) gespeicherte IMD-Ankerpunkt ist.

4. ED (102) nach einem der Ansprüche 1 bis 3, ferner umfassend:
einen oder mehrere Puffer (330); und
wobei die mindestens eine Steuerung (302, 324) ferner dazu konfiguriert ist, den einen oder die mehreren Puffer (330) zu steuern, um zu veranlassen, dass das weitere physiologische Signal und das physiologische Signal miteinander synchronisiert werden, wenn sie auf der Anzeige (314, 316), die die ED (102) aufweist oder die damit kommunikativ gekoppelt ist, gemeinsam angezeigt werden.

5. ED (102) nach einem der Ansprüche 1 bis 4, wobei:
das physiologische Signal eines von einem Elektrogramm, EGM, oder einem subkutanen Elektrokardiogramm, EKG, umfasst; und
das weitere physiologische Signal, das gemeinsam mit dem einen des EGM oder des subkutanen EKG angezeigt und synchronisiert ist, ein Elektrokardiogramm, EKG, umfasst, das unter Verwendung der einen oder der mehreren Hautelektroden (335) erfasst wird.

6. System (100), umfassend die ED (102) nach einem der Ansprüche 1 bis 5 und ferner umfassend die IMD (101), wobei die IMD (101) umfasst:
einen Speicher (250) der IMD (101);
einen Sendeempfänger (244) der IMD (101), der es der IMD (101) ermöglicht, drahtlos mit der ED (102) zu kommunizieren; und
mindestens eine Steuerung (221) der IMD (101), die mit dem Sendeempfänger (244) der IMD (101) und dem Speicher (250) der IMD (101) gekoppelt ist;
wobei, während die drahtlose Verbindung zwischen dem Sendeempfänger (326) der ED (102) und dem Sendeempfänger (244) der IMD (101) hergestellt wird, die mindestens eine Steuerung (221) der IMD (101) dazu konfiguriert ist:
Erhalten der physiologischen Signaldaten, die das physiologische Signal für den Patienten darstellen, in den die IMD (101) implantiert ist; und
Steuern des Sendeempfängers (244) der IMD (101), um die Mehrzahl von Datenpaketen an die ED (102) zu übertragen, wobei jedes Datenpaket der Mehrzahl von Datenpaketen, die durch die IMD (101) an die ED (102) übertragen werden, einen Abschnitt der physiologischen Signaldaten und einen jeweiligen Zeitstempel beinhaltet, der spezifiziert, wann das Datenpaket durch die IMD (101) aus einer Perspektive der IMD (101) übertragen wird.

7. System (100) nach Anspruch 6, wobei, während die drahtlose Verbindung im Herstellungsprozess zwischen dem Sendeempfänger (326) der ED (102) und dem Sendeempfänger (244) der IMD (101) ist, die mindestens eine Steuerung (221) der IMD (101) dazu konfiguriert ist, den Sendeempfänger (244) der IMD (101) dazu zu steuern, ein Advertising-Paket zu übertragen, das Verbindungsanforderungspaket von der ED (102) innerhalb eines Empfangsfensters zu empfangen, nachdem die IMD (101) das Advertising-Paket übertragen hat, und als Reaktion darauf ein Verbindungsantwortpaket, das den IMD-Ankerpunkt umfasst, an die ED (102) zu übertragen.

8. System nach einem der Ansprüche 6 oder 7, wobei:
die IMD (101) eine Echtzeituhr, RTC, (245) umfasst;
der IMD-Ankerpunkt einen Wert der RTC (245) der IMD (101) umfasst, wenn ein Verbindungsantwortpaket durch die IMD (101) als Reaktion darauf übertragen wird, dass die IMD (101) das Verbindungsanforderungspaket von der ED (102) empfängt; und
der jeweilige Zeitstempel jedes Datenpakets der Datenpakete, die durch die ED (102) von der IMD (101) empfangen werden, einen jeweiligen Wert der RTC (245) der IMD (101) umfasst, wenn das Datenpaket durch die IMD (101) übertragen wird.

9. System nach Anspruch 8, wobei:
die ED (102) eine Echtzeituhr, RTC, (345) umfasst; und
der ED-Ankerpunkt einen Wert der RTC (345) der ED (102) umfasst, wenn die ED (102) das Verbindungsantwortpaket von der IMD (101) empfängt.

10. System nach einem der Ansprüche 6 bis 9, wobei:
die physiologischen Signaldaten, die durch die IMD (101) erhalten werden, basierend auf einem physiologischen Echtzeitsignal bestimmt werden, das durch die IMD (101) oder durch eine weitere implantierbare Vorrichtung, die kommunikativ mit der IMD (101) gekoppelt ist, erfasst wird;
die weiteren physiologischen Signaldaten, die durch die ED (102) erhalten werden, basierend auf einem weiteren physiologischen Echtzeitsignal bestimmt werden, das durch die ED (102) oder durch eine weitere externe Vorrichtung, die kommunikativ mit der ED (102) gekoppelt ist, erfasst wird; und
die jeweiligen Segmente des physiologischen Signals und die weiteren physiologischen Signale in Echtzeit oder nahezu Echtzeit derart gemeinsam angezeigt werden, dass sie miteinander synchronisiert sind.

11. Verfahren zum synchronen Anzeigen von Signalsegmenten, wobei das Verfahren umfasst: Übertragen eines Verbindungsanforderungspakets durch eine externe Vorrichtung, ED, (102), an eine implantierbare medizinische Vorrichtung, IMD, (101) als Reaktion auf das Empfangen eines Advertising-Pakets von der IMD (101), um dadurch das Herstellen einer drahtlosen Verbindung zwischen der ED (102) und der IMD (101) zu ermöglichen;
Empfangen eines IMD-Ankerpunkts durch die ED (102) während eines Herstellungsprozesses der drahtlosen Verbindung zwischen der ED (102) und der IMD (101), der durch die IMD (101) als Reaktion darauf übertragen wird, dass die IMD (101) das Verbindungsanforderungspaket empfängt;
wobei die ED (102) den IMD-Ankerpunkt und einen ED-Ankerpunkt speichert;
Empfangen einer Mehrzahl von Datenpaketen durch die ED (102), während die drahtlose Verbindung zwischen der ED (102) und der IMD (101) hergestellt wird, wobei jedes davon einen Abschnitt physiologischer Signaldaten für einen Patienten, die durch die IMD (101) erhalten werden, und einen jeweiligen IMD-Zeitstempel umfasst, der spezifiziert, wann das Datenpaket durch die IMD (101) übertragen wird;
Bestimmen einer jeweiligen Zeit, zu der das Datenpaket durch die IMD (101) aus einer Perspektive der ED (102) übertragen wurde, durch die ED (102) für jedes Datenpaket, das durch die ED (102) empfangen wird, basierend auf dem ED-Ankerpunkt, der durch die ED (102) gespeichert ist, dem IMD-Ankerpunkt, der durch die ED (102) gespeichert ist, und dem IMD-Zeitstempel, der in dem durch die ED (102) empfangenen Datenpaket umfasst ist;
Erhalten weiterer physiologischer Signaldaten durch die ED (102), die ein weiteres physiologisches Signal darstellen, das unter Verwendung einer oder mehrerer Hautelektroden oder eines nicht implantierten Sensors erfasst wird; und
Verwenden der jeweiligen Zeiten durch die ED (102), die für die von der IMD (101) durch die ED (102) empfangenen Datenpakete bestimmt sind, um ein gemeinsames Anzeigen jeweiliger Segmente eines physiologischen Signals zu veranlassen, die durch die Datenpakete dargestellt sind, zusammen mit dem weiteren physiologischen Signal, sodass sie miteinander synchronisiert werden.

12. Verfahren nach Anspruch 11, wobei das Bestimmen der jeweiligen Zeiten durch die ED (102), zu denen ein das Datenpaket durch die IMD (101) aus der Perspektive der ED (102) übertragen wurde, durch die ED (102) für jedes Datenpaket der Datenpakete, die durch die ED (102) empfangen werden, unter Verwendung einer folgenden Gleichung durchgeführt wird:${RT}_{DATA} = {ANCHOR}_{ED} + {TIME_STAMP}_{IMD} - {ANCHOR}_{IMD} ,$ wobei
RT_{DATA} die jeweilige Zeit ist, zu der das Datenpaket durch die IMD (101) aus der Perspektive der ED (102) übertragen wurde, wobei die jeweilige Zeit durch die ED (102) bestimmt wird,
ANCHOR_{ED} der ED-Ankerpunkt ist, der durch die ED (102) gespeichert ist,
TIME_STAMP_{IMD} der IMD-Zeitstempel ist, der in dem Datenpaket umfasst ist, das von der ED (102) empfangen wird, und
ANCHOR_{IMD} der durch die ED (102) gespeicherte IMD-Ankerpunkt ist.

13. Verfahren nach einem der Ansprüche 11 oder 12, wobei:
die IMD (101) und die ED (102) jeweils eine jeweilige Echtzeituhr, RTC (245, 345), umfassen;
der IMD-Ankerpunkt, der von der ED (102) empfangen wird, einen Wert der RTC (245) der IMD (101) umfasst, wenn ein Verbindungsantwortpaket durch die IMD (101) als Reaktion darauf übertragen wird, dass die IMD (101) das Verbindungsanforderungspaket von der ED (102) empfängt; und
der jeweilige Zeitstempel jedes Datenpakets der Datenpakete, die durch die ED (102) von der IMD (101) empfangen werden, einen Wert der RTC (245) der IMD (101) umfasst, wenn das Datenpaket durch die IMD (101) übertragen wird.

14. Verfahren nach Anspruch 13, wobei:
der ED-Ankerpunkt einen Wert der RTC (345) der ED (102) umfasst, wenn die ED das Verbindungsantwortpaket von der IMD (101) empfängt.

15. Verfahren nach einem der Ansprüche 11 bis 14, wobei
die physiologischen Signaldaten, die durch die IMD (101) erhalten werden, basierend auf einem physiologischen Echtzeitsignal bestimmt werden, das durch die IMD (101) oder durch eine weitere implantierbare Vorrichtung, die kommunikativ mit der IMD (101) gekoppelt ist, erfasst wird;
die weiteren physiologischen Signaldaten, die durch die ED (102) erhalten werden, basierend auf einem weiteren physiologischen Echtzeitsignal bestimmt werden, das durch die ED (102) oder durch eine weitere externe Vorrichtung, die kommunikativ mit der ED (102) gekoppelt ist, erfasst wird; und
die jeweiligen Segmente des physiologischen Signals und die weiteren physiologischen Signale in Echtzeit oder nahezu Echtzeit derart gemeinsam angezeigt werden, dass sie miteinander synchronisiert sind.

## Revendications

1. Dispositif externe, ED, (102) configuré pour communiquer sans fil avec un dispositif médical implantable, IMD, (101), l'ED (102) comprenant :
une mémoire (330) ;
un émetteur-récepteur (326) qui permet à l'ED (102) de communiquer sans fil avec l'IMD (101) ; et
au moins un dispositif de commande (302, 324) relié à l'émetteur-récepteur (326) et à la mémoire (330) ;
dans lequel, pendant qu'une liaison sans fil est en cours d'établissement entre l'ED (102) et l'IMD (101), ledit au moins un dispositif de commande (302, 324) est configuré pour :
commander l'émetteur-récepteur (326) pour transmettre un paquet de demande de liaison à l'IMD (101) en réponse à la réception d'un paquet d'annonce provenant de l'IMD (101) afin de permettre ainsi d'établir la liaison sans fil entre l'ED (102) et l'IMD (101) ;
commander l'émetteur-récepteur (326) pour recevoir de l'IMD (101) un point d'ancrage d'IMD transmis par l'IMD (101) en réponse à la réception par l'IMD (101) du paquet de demande de liaison provenant de l'ED (102) ;
sauvegarder le point d'ancrage d'IMD dans la mémoire (330) ; et
déterminer un point d'ancrage d'ED et sauvegarder également le point d'ancrage d'ED dans la mémoire (330) ; et
dans lequel, pendant que la liaison sans fil est établie entre l'ED (102) et l'IMD (101), ledit au moins un dispositif de commande (302, 324) est configuré pour :
commander l'émetteur-récepteur (326) pour recevoir au moins une partie d'une pluralité de paquets de données provenant de l'IMD (101), dans lequel chaque paquet de données de la pluralité de paquets de données reçus de l'IMD (101) comprend une portion de données de signal physiologique et un horodatage respectif inclus par l'IMD (101) ;
déterminer, pour chaque dit paquet de données parmi les paquets de données reçus de l'IMD (101), un instant respectif auquel le paquet de données a été transmis par l'IMD (101) du point de vue de l'ED (102) sur la base du point d'ancrage d'ED qui est stocké dans la mémoire (330), du point d'ancrage d'IMD qui est stocké dans la mémoire (330), et de l'horodatage respectif inclus dans le paquet de données reçu de l'IMD (101) ;
obtenir des données de signal physiologique supplémentaires représentatives d'un signal physiologique supplémentaire détecté à l'aide d'une ou plusieurs électrodes cutanées (335) ou d'un capteur non implanté ; et
utiliser les instants respectifs, déterminés pour les paquets de données reçus de l'IMD (101), pour provoquer le co-affichage de segments respectifs d'un signal physiologique qui sont représentés par les paquets de données avec le signal physiologique supplémentaire de sorte qu'ils soient synchronisés les uns avec les autres sur un affichage (314, 316) de l'ED (102) ou relié en communication à ce dernier.

2. Dispositif externe (102) selon la revendication 1, dans lequel le point d'ancrage d'IMD et le point d'ancrage d'ED correspondent à l'instant auquel une liaison sans fil est établie entre l'ED (102) et l'IMD (101).

3. Dispositif externe (102) selon l'une quelconque des revendications 1 ou 2, dans lequel ledit au moins un dispositif de commande (302, 324) est configuré pour déterminer l'instant respectif auquel un dit paquet de données a été transmis par l'IMD (101) du point de vue de l'ED (102), pour chaque dit paquet de données des paquets de données reçus par l'ED (102), en utilisant l'équation suivante :${RT}_{DATA} = {ANCHOR}_{ED} + {TIME_STAMP}_{IMD} - {ANCHOR}_{IMD} ,$ où
RT_{DATA} est l'instant respectif auquel le paquet de données a été transmis par l'IMD (101) du point de vue de l'ED (102), lequel instant respectif est déterminé par l'ED (102),
ANCHOR_{ED} est le point d'ancrage d'ED stocké par l'ED (102),
TIME_STAMP_{IMD} est l'horodatage d'IMD inclus dans le paquet de données reçu par l'ED (102), et
ANCHOR_{IMD} est le point d'ancrage d'IMD stocké par l'ED (102).

4. Dispositif externe (102) selon l'une quelconque des revendications 1 à 3, comprenant en outre :
un ou plusieurs tampons (330) ; et
dans lequel ledit au moins un dispositif de commande (302, 324) est en outre configuré pour commander ledit un ou plusieurs tampons (330) pour faire en sorte que le signal physiologique supplémentaire et le signal physiologique soient synchronisés l'un avec l'autre lorsqu'ils sont co-affichés sur l'affichage (314, 316) de l'ED (102) ou relié en communication à ce dernier.

5. Dispositif externe (102) selon l'une quelconque des revendications 1 à 4, dans lequel :
le signal physiologique comprend soit un électrogramme, EGM, soit un électrocardiogramme sous-cutané, ECG ; et
le signal physiologique supplémentaire, qui est co-affiché et synchronisé avec ledit EGM ou ledit ECG sous-cutané, comprend un électrocardiogramme, ECG, détecté à l'aide desdites une ou plusieurs électrodes cutanées (335).

6. Système (100) comprenant l'ED (102) selon l'une quelconque des revendications 1 à 5, et comprenant en outre l'IMD (101), dans lequel l'IMD (101) comprend :
une mémoire (250) de l'IMD (101) ;
un émetteur-récepteur (244) de l'IMD (101) qui permet à l'IMD (101) de communiquer sans fil avec l'ED (102) ; et
au moins un dispositif de commande (221) de l'IMD (101) relié à l'émetteur-récepteur (244) de l'IMD (101) et à la mémoire (250) de l'IMD (101) ;
dans lequel, pendant que la liaison sans fil est établie entre l'émetteur-récepteur (326) de l'ED (102) et l'émetteur-récepteur (244) de l'IMD (101), ledit au moins un dispositif de commande (221) de l'IMD (101) est configuré pour :
obtenir les données de signal physiologique représentatives du signal physiologique pour le patient dans lequel l'IMD (101) est implanté ; et
commander l'émetteur-récepteur (244) de l'IMD (101) pour transmettre la pluralité de paquets de données à l'ED (102), dans lequel chaque paquet de données, de la pluralité de paquets de données qui sont transmis par l'IMD (101) à l'ED (102), comprend une portion des données de signal physiologique et un horodatage respectif spécifiant l'instant auquel le paquet de données est transmis par l'IMD (101) du point de vue de l'IMD (101).

7. Système (100) selon la revendication 6, dans lequel, pendant que la liaison sans fil est en cours d'établissement entre l'émetteur-récepteur (326) de l'ED (102) et l'émetteur-récepteur (244) de l'IMD (101), ledit au moins un dispositif de commande (221) de l'IMD (101) est configuré pour commander l'émetteur-récepteur (244) de l'IMD (101) pour transmettre un paquet d'annonce, recevoir le paquet de demande de liaison de l'ED (102) dans une fenêtre de réception faisant suite à l'instant auquel l'IMD (101) transmet le paquet d'annonce, et en réponse à cela, transmettre à l'ED (102) un paquet de réponse de liaison comprenant le point d'ancrage d'IMD.

8. Système selon l'une quelconque des revendications 6 ou 7, dans lequel :
l'IMD (101) comprend une horloge en temps réel, RTC (245) ;
le point d'ancrage d'IMD comprend une valeur de la RTC (245) de l'IMD (101) lorsqu'un paquet de réponse de liaison est transmis par l'IMD (101) en réponse à la réception par l'IMD (101) du paquet de demande de liaison provenant de l'ED (102) ; et
l'horodatage respectif de chaque dit paquet de données parmi les paquets de données reçus par l'ED (102) à partir de l'IMD (101) comprend une valeur respective de la RTC (245) de l'IMD (101) lorsque le paquet de données est transmis par l'IMD (101).

9. Système selon la revendication 8, dans lequel :
l'ED (102) comprend une horloge en temps réel, RTC (345) ; et
le point d'ancrage d'ED comprend une valeur de la RTC (345) de l'ED (102) lorsque l'ED (102) reçoit, de l'IMD (101), le paquet de réponse de liaison.

10. Système selon l'une quelconque des revendications 6 à 9, dans lequel :
les données de signal physiologique, qui sont obtenues par l'IMD (101), sont déterminées sur la base d'un signal physiologique en temps réel qui est détecté par l'IMD (101) ou par un dispositif implantable supplémentaire relié en communication à l'IMD (101) ;
les données de signal physiologique supplémentaires, qui sont obtenues par l'ED (102), sont déterminées sur la base d'un signal physiologique en temps réel supplémentaire qui est détecté par l'ED (102) ou par un dispositif externe supplémentaire relié en communication à l'ED (102) ; et
les segments respectifs du signal physiologique et les signaux physiologiques supplémentaires sont co-affichés en temps réel ou quasi en temps réel de sorte qu'ils soient synchronisés les uns avec les autres.

11. Procédé pour l'affichage synchrone de segments de signaux, le procédé comprenant : la transmission, par un dispositif externe, ED (102), d'un paquet de demande de liaison à un dispositif médical implantable, IMD, (101) en réponse à la réception d'un paquet d'annonce de l'IMD (101) afin de permettre ainsi l'établissement d'une liaison sans fil entre l'ED (102) et l'IMD (101) ;
la réception, par l'ED (102), pendant un processus d'établissement de la liaison sans fil entre l'ED (102) et l'IMD (101), d'un point d'ancrage d'IMD transmis par l'IMD (101) en réponse à la réception par l'IMD (101) du paquet de demande de liaison ;
le stockage, par l'ED (102), du point d'ancrage d'IMD et d'un point d'ancrage d'ED ;
la réception, par l'ED (102), pendant que la liaison sans fil est établie entre l'ED (102) et l'IMD (101), d'une pluralité de paquets de données, chacun d'eux comprenant une portion de données de signal physiologique pour un patient obtenues par l'IMD (101) et un horodatage d'IMD respectif spécifiant l'instant auquel le paquet de données est transmis par l'IMD (101) ;
la détermination, par l'ED (102), pour chaque dit paquet de données reçu par l'ED (102), d'un instant respectif auquel le paquet de données a été transmis par l'IMD (101) du point de vue de l'ED (102) sur la base du point d'ancrage d'ED qui est stocké par l'ED (102), du point d'ancrage d'IMD qui est stocké par l'ED (102), et de l'horodatage d'IMD inclus dans le paquet de données reçu par l'ED (102) ;
l'obtention, par l'ED (102), de données de signal physiologique supplémentaires représentatives d'un signal physiologique supplémentaire détecté à l'aide d'une ou plusieurs électrodes cutanées ou d'un capteur non implanté ; et
l'utilisation, par l'ED (102), des instants respectifs, déterminés pour les paquets de données reçus par l'ED (102) depuis l'IMD (101), pour provoquer le co-affichage de segments respectifs d'un signal physiologique qui sont représentés par les paquets de données avec le signal physiologique supplémentaire de sorte qu'ils soient synchronisés les uns avec les autres.

12. Procédé selon la revendication 11, dans lequel la détermination par l'ED (102) de l'instant respectif auquel un dit paquet de données a été transmis par l'IMD (101) du point de vue de l'ED (102), est exécutée par l'ED (102) pour chaque dit paquet de données des paquets de données reçus par l'ED (102), en utilisant l'équation suivante :${RT}_{DATA} = {ANCHOR}_{ED} + {TIME_STAMP}_{IMD} - {ANCHOR}_{IMD} ,$ où
RT_{DATA} est l'instant respectif auquel le paquet de données a été transmis par l'IMD (101) du point de vue de l'ED (102), lequel instant respectif est déterminé par l'ED (102),
ANCHOR_{ED} est le point d'ancrage d'ED stocké par l'ED (102),
TIME_STAMP_{IMD} est l'horodatage d'IMD inclus dans le paquet de données reçu par l'ED (102), et
ANCHOR_{IMD} est le point d'ancrage d'IMD stocké par l'ED (102).

13. Procédé selon l'une quelconque des revendications 11 ou 12, dans lequel :
l'IMD (101) et l'ED (102) comprennent chacun une horloge en temps réel respective, RTC (245, 345) ;
le point d'ancrage d'IMD reçu par l'ED (102) comprend une valeur de la RTC (245) de l'IMD (101) lorsqu'un paquet de réponse de liaison est transmis par l'IMD (101) en réponse à la réception par l'IMD (101) du paquet de demande de liaison provenant de l'ED (102) ; et
l'horodatage respectif de chaque dit paquet de données parmi les paquets de données reçus par l'ED (102) à partir de l'IMD (101) comprend une valeur de la RTC (245) de l'IMD (101) lorsque le paquet de données est transmis par l'IMD (101).

14. Procédé selon la revendication 13, dans lequel :
le point d'ancrage d'ED comprend une valeur de la RTC (345) de l'ED (102) lorsque l'ED reçoit, de l'IMD (101), le paquet de réponse de liaison.

15. Procédé selon l'une quelconque des revendications 11 à 14, dans lequel :
les données de signal physiologique, qui sont obtenues par l'IMD (101), sont déterminées sur la base d'un signal physiologique en temps réel qui est détecté par l'IMD (101) ou par un dispositif implantable supplémentaire relié en communication à l'IMD (101) ;
les données de signal physiologique supplémentaires, qui sont obtenues par l'ED (102), sont déterminées sur la base d'un signal physiologique en temps réel supplémentaire qui est détecté par l'ED (102) ou par un dispositif externe supplémentaire relié en communication à l'ED (102) ; et
les segments respectifs du signal physiologique et les signaux physiologiques supplémentaires sont co-affichés en temps réel ou quasi en temps réel de sorte qu'ils soient synchronisés les uns avec les autres.
